(19) Europäisches Patentamt
European Patent Office
Office européen des brevets

(11) **EP 4 368 097 B1**

(12) **EUROPEAN PATENT SPECIFICATION**

(45) Date of publication and mention
of the grant of the patent:
**17.06.2026 Bulletin 2026/25**

(21) Application number: **22206814.0**

(22) Date of filing: **10.11.2022**

(51) International Patent Classification (IPC):
**A61B 5/00** *(2006.01)*     **A61B 5/145** *(2006.01)*
**A61B 5/1455** *(2006.01)*     **G16H 50/20** *(2018.01)*

(52) Cooperative Patent Classification (CPC):
**A61B 5/0095; A61B 5/0093; A61B 5/14532;
A61B 5/1455; A61B 5/7264; A61B 5/7275;
G16H 50/20;** A61B 5/7267

(54) **PROCESS FOR NON-INVASIVELY ANALYZING A MULTILAYER MEDIUM**

VERFAHREN ZUR NICHTINVASIVEN ANALYSE EINES MEHRSCHICHTIGEN MEDIUMS

PROCÉDÉ D'ANALYSE NON INVASIVE D'UN MILIEU MULTICOUCHE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**15.05.2024 Bulletin 2024/20**

(73) Proprietor: **Eclypia
38000 Grenoble (FR)**

(72) Inventors:
• **MONPEURT, Cyrielle
38000 GRENOBLE (FR)**
• **BLANC, Romain
38000 GRENOBLE (FR)**
• **GALLEGOS, Alexandre
75002 PARIS (FR)**

(74) Representative: **Fidal Innovation
4-6 avenue d'Alsace
92400 Courbevoie (FR)**

(56) References cited:
**CN-A- 109 662 720     US-A1- 2016 374 565**

• **HU HANPING ET AL: "Generalized theory of the
photoacoustic effect in a multilayer material",
JOURNAL OF APPLIED PHYSICS, AMERICAN
INSTITUTE OF PHYSICS, 2 HUNTINGTON
QUADRANGLE, MELVILLE, NY 11747, vol. 86, no.
7, 1 October 1999 (1999-10-01), pages 3953 -
3958, XP012048746, ISSN: 0021-8979, DOI:
10.1063/1.371313**

**Description**

**FIELD OF THE INVENTION**

The present invention relates to a process for non-invasively analyzing a target multilayer medium and to the corresponding non-invasive sensor.

More specifically, the invention relates to a process for determining the sense of variation of a parameter of interest with an amplitude and/or a phase of a wave detected with a non-invasive sensor based on the detection of a photothermal or photoacoustic effect, in particular in a multilayer target medium, the structure of which can evolve over time. A parameter of interest may for example be blood glucose in the skin.

**TECHNOLOGICAL BACKGROUND**

In the field of sensors for living organisms, non-invasive sensors based on photoacoustic or photothermal detection are known.

An area of interest of a medium to be analyzed, called target, is irradiated by means of a laser beam of wavelength(s) and modulation frequency(ies) chosen according to the parameter of interest to be measured.

The laser beam is absorbed by the target over a characteristic length that depends on the structure of the target. The absorption of light energy leads to local heating of the target. In response to this heating, a thermal wave of frequency equal to the modulation frequency of the laser is generated in the target. This thermal wave propagates in the target and in particular up to the outer surface of the target.

The thermal wave can be directly detected and analyzed. In this case, we speak of "photothermal detection".

Photoacoustic detection exploits the fact that the thermal wave is associated with a pressure wave having a frequency identical to the modulation frequency of the laser.

In the case of "indirect photoacoustic detection", the pressure wave generated in the fluid external environment is detected when the thermal wave generated in the target reaches, after propagation, the target interface - fluid external environment.

The photoacoustic effect has been the subject of numerous theoretical studies. Allan Rosencwaig and Allen Gersho have developed a theoretical model of the photoacoustic signal. This model involves the physico-chemical properties of the sample to be analyzed, including the optical scattering length, the thickness and the thermal scattering length of the sample.

(Rosencwaig, A. and Gersho, A. (1976), Theory of the photoacoustic effect with solids, Journal of Applied Physics, 47, 64).

Hu et al. developed a generalized theory of the photoacoustic effect in a laminated material.

(Hu, H., Wang, X., & Xu, X. (1999). Generalized theory of the photoacoustic effect in a multilayer material. Journal of Applied Physics, 86, 3953-3958.)

Photoacoustic detection has many advantages over other detection techniques, including the orthogonal aspect of transduction: the optical signal at the input of the medium to be analyzed is converted into an acoustic signal :

- that is very specific to the phenomenon to be observed,
- and that allows the use of inexpensive and miniaturized sensors.

The difficulty of photoacoustic or photothermal detection stems, among other things:

- from the number of parameters generally influencing the detected signal and,
- for certain analytes of interest present in low concentration in the medium to be analyzed, from the small proportion of the detected signal specific to each of these parameters of interest.

Photoacoustic detection or photothermal detection requires not only a choice of wavelength(s) but also a choice of the modulation frequency(ies) of the laser to be used. Indeed, the characteristic length of penetration of the incident light wave into the target depends on the modulation frequency of the laser. If the structure of the target (e.g. skin) changes over time, the modulation frequency(ies) to be used to measure the parameter of interest (e.g. interstitial blood glucose) with controlled accuracy and consumption also changes over time.

In addition, in a stratified target medium, the reflections at the interfaces between the different layers give rise from a so-called "primary" thermal wave propagating towards the surface of the target medium to a multitude of waves, called "secondary", "tertiary", etc., which are likely to interfere with each other, as shown by many authors. Thermal wave interferences in the context of photothermal effect has been described in the following documents:

- C. A. Bennett and R. R. Patty, "Thermal wave interferometry: a potential application of the photoacoustic effect," Appl. Opt. 21, 49-54 (1982);
- Andreas Mandelis, "Theory of photothermal-wave diffraction and interference in condensed media," J. Opt. Soc. Am. A 6, 298-308 (1989) ;
- Andreas Mandelis and Kwan F. Leung, "Photothermal-wave diffraction and interference in condensed media: experimental evidence in aluminum," J. Opt. Soc. Am. A 8, 186-200 (1991). This interference phenomenon is likely to affect the accuracy of a non-invasive sensor based on photoacoustic detection. In a stratified target medium the structure of which is likely to evolve, the interference conditions are also likely to change since they depend on the structure of the target medium. As a result, interference conditions are not constant over time.

CN 109662720 A relates to the field of non-invasive blood glucose detection, in particular to a multi-band photoacoustic non-invasive blood glucose concentration prediction system based on deep learning.

Depending on the modulation frequency and on the structure of the stratified target medium (skin in the case of glycemia), the resulting thermal wave that is detected can therefore correspond to more or less constructive or destructive interferences and thus be negatively or positively correlated to the parameter to be measured, rendering difficult to assess whether a variation of the thermal wave that is detected can be associated with an increase of the parameter to be measured or a decrease of this parameter.

The invention therefore aims at proposing a process for non-invasively analyzing a parameter of interest in a target multilayer medium comprising the determination of the sense of variation of at least one of the parameters to be analyzed with the variations of at least one characteristic of the detected signal among an amplitude and a phase, in the context of a non-invasive sensor based on photoacoustic or photothermal detection.

## SUMMARY OF THE INVENTION

Hence, the invention relates to a process for analyzing at least one parameter of interest in a target multilayer medium with a non-invasive sensor based on photoacoustic or photothermal detection, the process comprising:

a) providing a non-invasive sensor comprising :

- a light source, the irradiation configuration of which is tunable, this irradiation configuration comprising at least one wavelength and at least one respective associated intensity modulation frequency,
- a detection cell configured for detecting an acoustic or thermal signal,
- a signal processing module,
- an adaptation module comprising a processor and a memory storing an abacus of correlation cases, the abacus of correlation cases comprising, for each of a plurality of pairs comprising one of a plurality of model configuration of the target multilayer medium (2) and one of a plurality of irradiation configuration of the light source, at least one associated correlation case which comprises for at least one wavelength of the irradiation configuration and a respective associated modulation frequency:

  - a first sign which is the sign of the correlation coefficient between the amplitude of the detected wave at the respective wavelength and associated modulation frequency and the parameter of interest,
  - a second sign which is the sign of the correlation coefficient between the phase of the detected wave at the respective wavelength and associated modulation frequency and the parameter of interest, and
  - a third sign which is the sign of the correlation coefficient between the amplitude and the phase of the detected wave at the respective wavelength and associated modulation frequency;

b) determining a particular model configuration of the target multilayer medium;

c) determining with the adaptation module a particular irradiation configuration for subsequent irradiation based on the abacus of correlation cases and on the particular model configuration ;

d) performing a plurality of irradiations of the target multilayer medium during an irradiation time interval with the light source configured with the particular irradiation configuration determined at step c) and detecting the respective signal generated in response to the irradiation with the detection cell and determining a respective amplitude and a respective phase of said signal at at least one wavelength and at least one respective associated modulation frequency of the particular irradiation configuration with the signal processing module;

e) with the adaptation module, calculating a third sign of a correlation case associated with at least one of the at least one wavelength and at least one respective associated modulation frequency of the particular irradiation configuration based on the determined respective amplitudes and phases;

f) determining with the adaptation module a correlation case associated with at least one of the at least one wavelength and the respective modulation frequency of the particular irradiation configuration based on the abacus of correlation cases and the respective calculated third sign;

g) determining with the adaptation module the sense of variation of at least one parameter of interest with an amplitude and/or a phase of the thermal or acoustic signal based on the at least one associated correlation case determined at f).

Such a process makes it possible to determine the sense of variation of at least one parameter of interest with the amplitude and/or the phase of the thermal or acoustic signal at at least one wavelength and an associated modulation frequency without prior measurements or without prior knowledge of the structure of the multilayer target except parameter ranges (or equivalently domain) for the level 1 parameters and/or for the at least one parameter of interest.

This information can be useful to determine the sense of variation of a parameter of interest over time, in particular with reduced power and time consumption and/or to estimate the confidence level the user can have in the very non-invasive sensor on which the process is carried out or another complementary non-invasive sensor.

In a particular embodiment, the process for analyzing at least one parameter of interest in a target multilayer medium comprises after g) : determining the sense of variation over time of at least one parameter of interest based on the signal generated in response to an additional irradiation of the target multilayer medium (2) with the particular irradiation configuration and the sense of variation with the amplitude and/or the phase of the thermal or acoustic signal determined at g). In this case, a non-invasive sensor on which the process is carried out can be used for example to raise alerts related to this sense of variation over time without requiring at least two successive measurements of the value of the parameter of interest, that are time and power consuming.

In a particular embodiment, the process for analyzing at least one parameter of interest in a target multilayer medium comprises after f) and before g) at least one iteration of a), b), c), d) , e) and f).

Such an embodiment allows to iteratively adapt the irradiation configuration according to the information obtained at the successive irradiations. This renders the determination of the sense of variation at step g) more accurate and/or reliable.

In an embodiment, the process for analyzing at least one parameter of interest in a target multilayer medium comprises before a) :

    i- performing an induced controlled variation of the parameter of interest,

    ii- performing an additional step c),

    iii- performing an additional step e),

    iv- with the adaptation module, calculating a first sign and a second sign of a correlation case associated with at least one of the at least one wavelength and at least one respective associated modulation frequency of the particular irradiation configuration determined at step ii) based on the respective amplitudes and phases determined at step iii),

    v- performing an additional step f),

and providing as additional input for a) the correlation case determined at v.

This embodiment allows one to obtain more knowledge on the multilayer medium prior to the first a), in the cases where the first determination of an irradiation configuration is not accurate enough or not possible with respect to the particular target multilayer medium. This could be necessary for example in case of a glycemia sensor where the patient's skin is very different from a "mean" skin of the state of the art.

The invention also relates to a non-invasive sensor for analyzing at least one parameter of interest in a target multilayer medium based on photoacoustic or photothermal detection comprising:

- a light source, the irradiation configuration of which is tunable, this irradiation configuration comprising at least one wavelength and at least one respective associated intensity modulation frequency;
- a detection cell configured for detecting an acoustic or thermal signal;
- a signal processing module;
- an adaptation module comprising :

    1) a processor,

    2) a memory storing an abacus of correlation cases,

the abacus of correlation cases comprising, for each of a plurality of pairs comprising one of a plurality of model configuration of the target multilayer medium and one of a plurality of irradiation configuration of the light source, at least one associated correlation case which comprises for at least one wavelength of the irradiation configuration and a respective associated modulation frequency:

- a first sign which is the sign of the correlation coefficient between the amplitude of the detected wave and the parameter of interest,
- a second sign which is the sign of the correlation coefficient between the phase of the detected wave and the parameter of interest, and
- a third sign which is the sign of the correlation coefficient between the amplitude and the phase of the detected wave at the respective wavelength

the adaptation module being configured for :

*determining a particular model configuration of the target multilayer medium,
*determining a particular irradiation configuration for subsequent irradiation based on the abacus of correlation cases and on the particular model configuration,
*calculating a third sign of a correlation case associated with at least one of the at least one wavelength and a respective associated modulation frequency of the particular irradiation configuration based on the determined respective amplitudes and phases,
*determining a correlation case associated with at least one of the at least one wavelength and a respective associated modulation frequency of a particular irradiation configuration based on the abacus of correlation cases and the respective calculated third sign and,
* determining a sense of variation of at least one parameter of interest with an amplitude and or a phase of a thermal or acoustic signal based on at least one determined correlation case.

The invention further deals with a computer program comprising instructions which, when executed, cause the non-invasive sensor of the preceding embodiments to execute the steps of the process according to any of the preceding embodiments.

Last, it is described the use of an abacus of correlation cases in a non-invasive sensor for analyzing at least one parameter of interest in a target multilayer medium based on photoacoustic or photothermal detection, the abacus of correlation cases comprising, for each of a plurality of pairs comprising one of a plurality of model configuration of the target multilayer medium (2) and one of a plurality of irradiation configuration of the light source, at least one associated correlation case which comprises for at least one wavelength of the irradiation configuration and at least one respective associated modulation frequency:

- a first sign which is the sign of the correlation coefficient between the amplitude of the detected wave at the respective wavelength and associated modulation frequency and the parameter of interest,
- a second sign which is the sign of the correlation coefficient ($Rparam/ph(\lambda, fmod)$) between the phase of the detected wave at the respective wavelength and associated modulation frequency and the parameter of interest, and
- a third sign which is the sign of the correlation coefficient ($Rph/amp(\lambda, fmod)$) between the amplitude and the phase of the detected wave at the respective wavelength and associated modulation frequency.

Indeed, such an abacus of correlation cases contains information on the correlation between a phase of the detected signal and the parameter of interest and on the correlation between an amplitude of the detected signal and the parameter of interest that can be useful to monitor the parameter of interest more accurately or in a more reliable manner.

## BRIEF DESCRIPTION OF THE DRAWINGS

Embodiments of the invention will be described below, in relation to the following drawings :

**Figure 1** represents the main elements of a non-invasive sensor 1 according to the invention, the non-invasive sensor 1 being in this case positioned in contact with a target multilayer medium.
**Figure 2** shows in a simplified case the different transmitted and reflected thermal waves generated in a target bilayer medium in response to irradiation by a laser (dashed arrow).
**Figure 3a** shows the impact on the interference phenomenon on the amplitude (upper graph) and the phase (lower graph) of the resulting thermal wave in the case of a skin model comprising two layers :

- an upper layer modelling the stratum corneum, this upper layer having a thickness of 18 $\mu$m (micrometers), a water content of 20% and no glucose;
- and a lower layer modelling the epidermis, having a water content of 60%, a glucose content equal respectively 50.0 mg/dL ; 162.5 mg/dL ; 275.0 mg/dL and 387.5 mg/dL (depending on the curve observed) and having an infinite thickness,

the parameter of interest being in this case the glucose content of the lower layer.

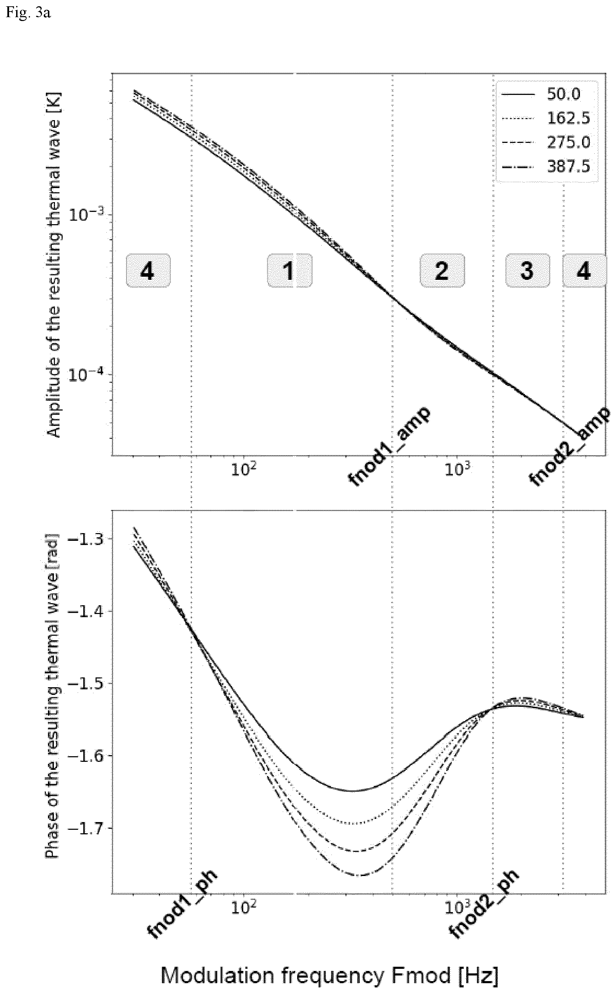

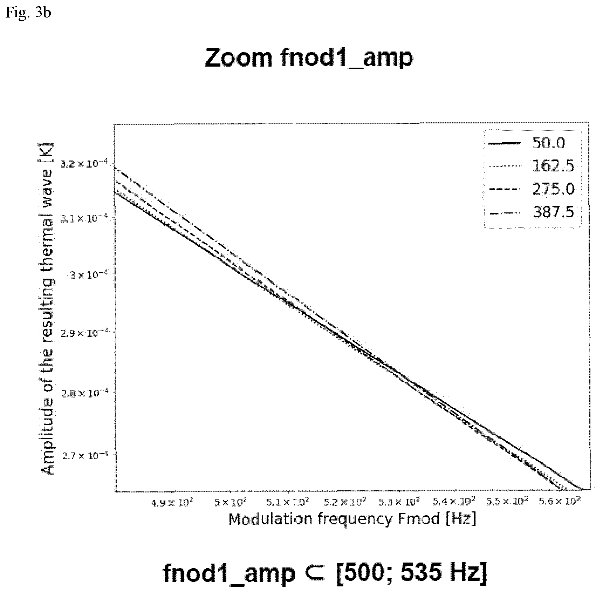

**Figure 3b** is a zoomed view of the graph of figure 3a for modulation frequencies between 480 Hz and 570 Hz.

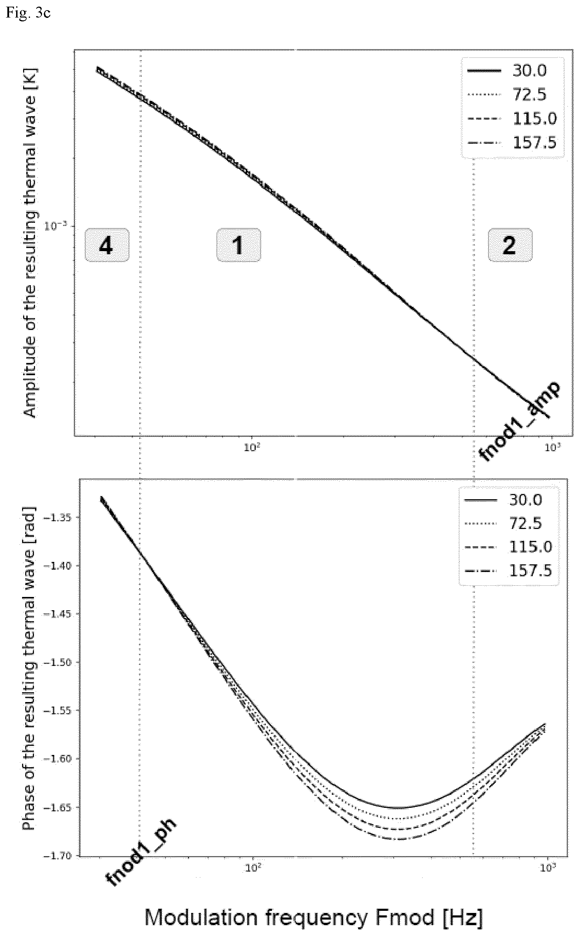

**Figure 3c** shows the impact on the interference phenomenon on the amplitude (upper graph) and the phase (lower graph) of the resulting thermal wave in the case of a skin model comprising two layers :

- an upper layer modelling the stratum corneum, this upper layer having a thickness of 18 $\mu$m (micrometers), a water content of 20% and no lactate;
- and a lower layer modelling the epidermis, having a water content of 60%, a glucose content equal respectively 30 mg/dL ; 72.50 mg/dL ; 115 mg/dL and 157.5 mg/dL (depending on the curve observed) and having an infinite thickness, the parameter of interest being in this case the lactate content of the lower layer.

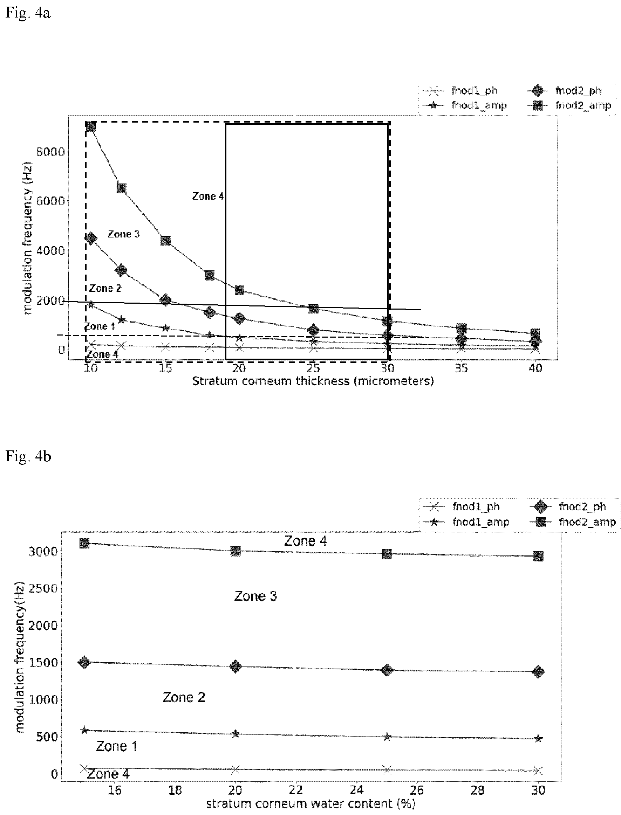

**Figure 4a** represents the two first nodal modulation frequencies for the amplitude of the thermal wave fnod_amp_1 and fnod_amp_2 and the two first nodal modulation frequencies for the phase of the thermal wave fnod_ph_1 and fnod_ph_2 as a function of the thickness of the upper layer in the case of a skin model comprising two layers :

- an upper layer modelling the stratum corneum, this upper layer having a thickness varying between 10 $\mu$m and 40 $\mu$m, a water content of 20% and no glucose;
- and a lower layer modelling the epidermis, having a water content of 60% and having an infinite thickness.

**Figure 4b** represents the two first nodal modulation frequencies for the amplitude of the thermal wave fnod_amp_1 and fnod_amp_2 and the two first nodal modulation frequencies for the phase of the thermal wave fnod_ph_1 and fnod_ph_2 as a function of the water content of the upper layer in the case of a skin model comprising two layers :

- an upper layer modelling the stratum corneum, this upper layer having a thickness of 18 $\mu$m, a water content varying from 15 % to 30% and no glucose;
- and a lower layer modelling the epidermis, having a water content of 60% and having an infinite thickness.

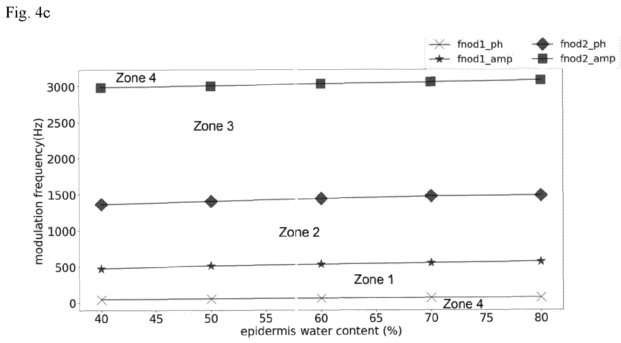

**Figure 4c** represents the two first nodal modulation frequencies for the amplitude of the thermal wave fnod _amp_1 and fnod_amp_2 and the two first nodal modulation frequencies for the phase of the thermal wave fnod_ph_1 and fnod_ph_2 as a function of the water content of the lower layer in the case of a skin model comprising two layers :

- an upper layer modelling the stratum corneum, this upper layer having a thickness of 18 $\mu$m, a water content of 20% and no glucose;
- and a lower layer modelling the epidermis, having a water content varying from 40% to 80% and having an infinite thickness.

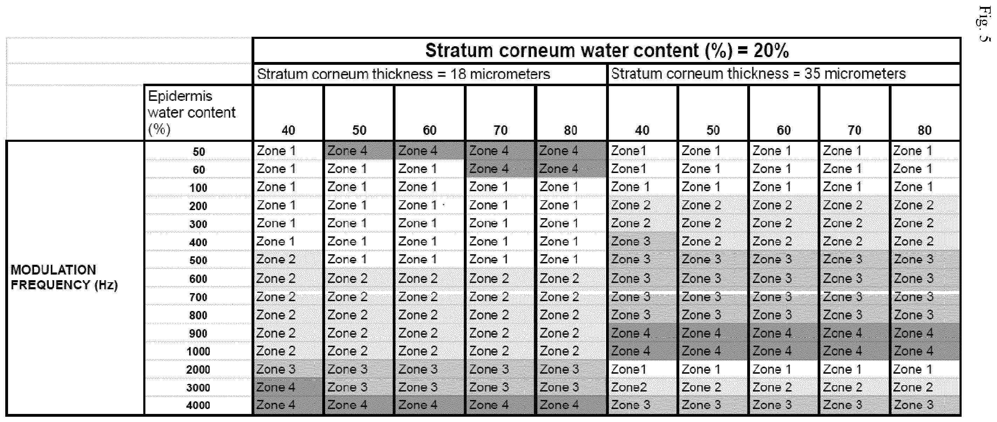

**Figure 5** is an abstract of an abacus of correlation cases, for associating a zone (or equivalently a correlation case) with a modulation frequency in the case of a skin model comprising two layers:

- an upper layer modelling the stratum corneum, this upper layer having a thickness either of 18 $\mu$m or of 35 $\mu$m, a water content of 20% and no glucose;
- and a lower layer modelling the epidermis, having a water content varying from 40% to 80% and having an infinite thickness.

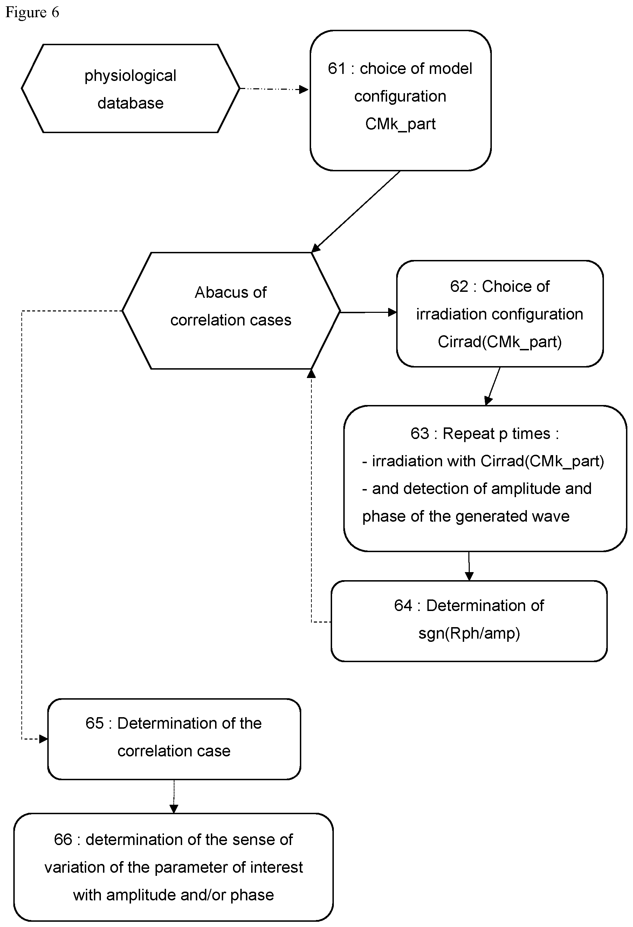

**Figure 6** is a flowchart of a first embodiment of the method according to the invention.

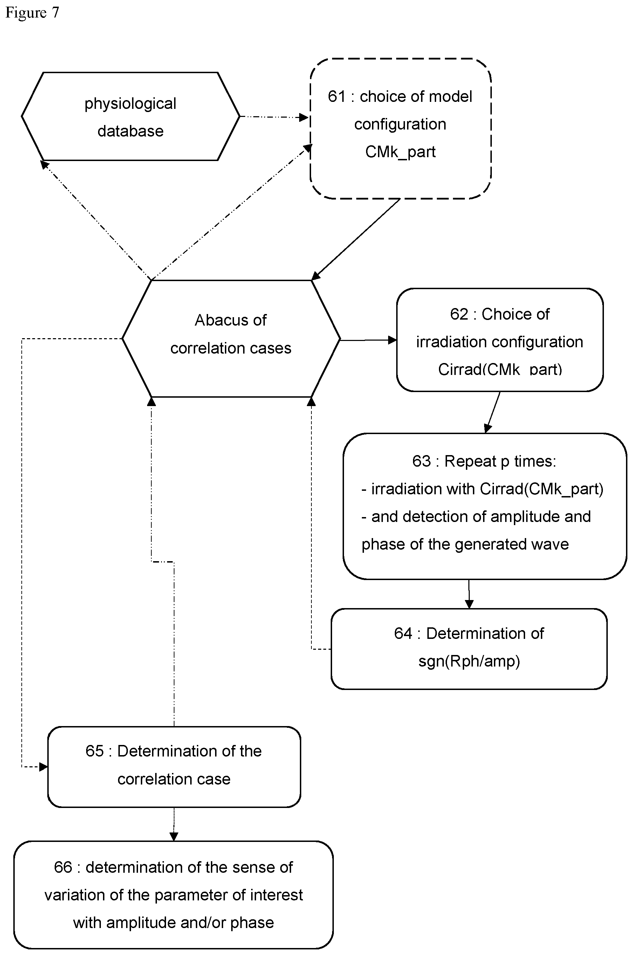

**Figure 7** is a flowchart of a second embodiment of the method according to the invention comprising an optional iteration of some steps, including the choice of the model configuration and of the irradiation configuration.

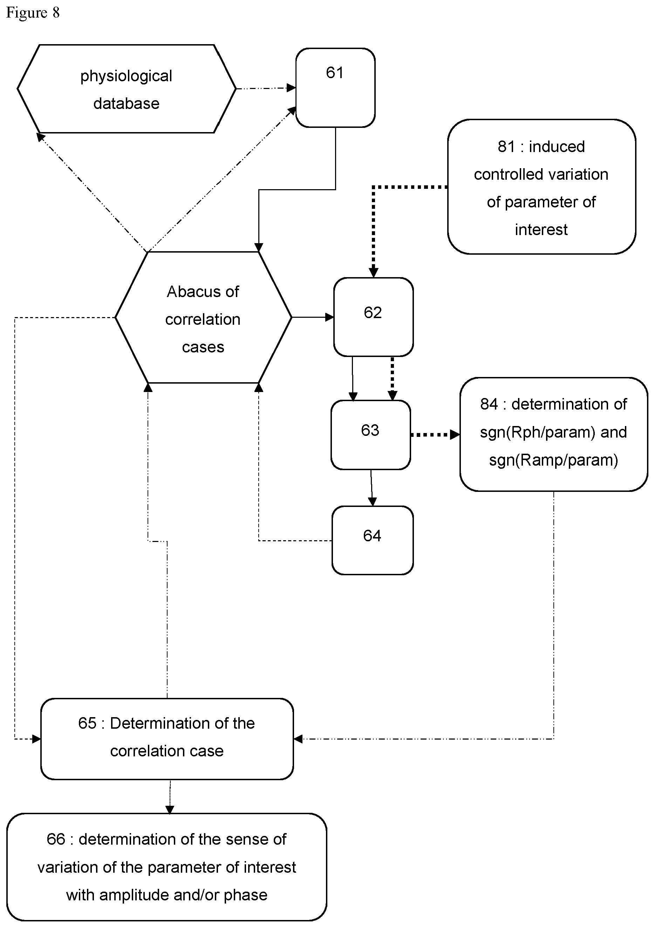

**Figure 8** is a flowchart of a third embodiment of the method according to the invention comprising, in addition to the flowchart of figure 7, optional steps for initializing the choice of the model configuration.

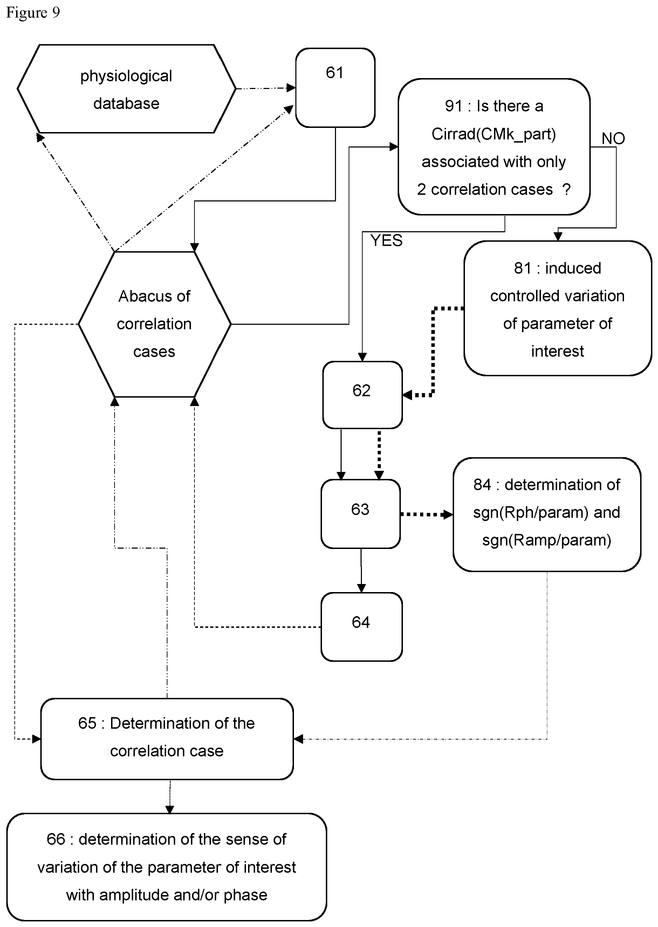

**Figure 9** is a flowchart of a fourth embodiment of the method according to the invention comprising, in addition to the flowchart of figure 8, a possible iteration of the steps for initializing the choice of the model configuration.

On the drawings, the same reference signs show the same or similar objects.

## DETAILED DESCRIPTION

The invention relates to a process to be implemented on a non-invasive sensor 1 configured for analyzing one or more parameters of a target multilayer medium 2, the structure of which possibly evolves over time. The target multilayer medium 2 may be, for example, a tissue of a human or animal organism, such as the skin.

The parameters to be measured are called hereafter "parameters of interest".

A parameter of interest may in particular be a physiological parameter in the case where the multilayer medium is a tissue of a human being or an animal.

For example, a physiological parameter to be measured is blood glucose, and more particularly interstitial blood glucose.

As non-limitative examples, a physiological parameter to be measured can also be the water content or the lactate concentration of a particular layer of the skin.

The non-invasive sensor 1 can be portable, and it can allow continuous monitoring of the parameter(s) of interest.

The non-invasive sensor 1 can be based on photoacoustic detection or photothermal detection. The process according to the invention is particularly useful in the case of a non-invasive sensor based on indirect photoacoustic detection, for which the non-invasive sensor 1 detects an acoustic wave generated in the fluid surrounding the target multilayer medium 2 in response to a laser irradiation. Nonetheless, it is also possible to implement this process on a non-invasive sensor based on photothermy.

To facilitate understanding, the example of indirect photoacoustic detection will be described in more detail below, but generalization to a sensor based on photothermy will be done without difficulty.

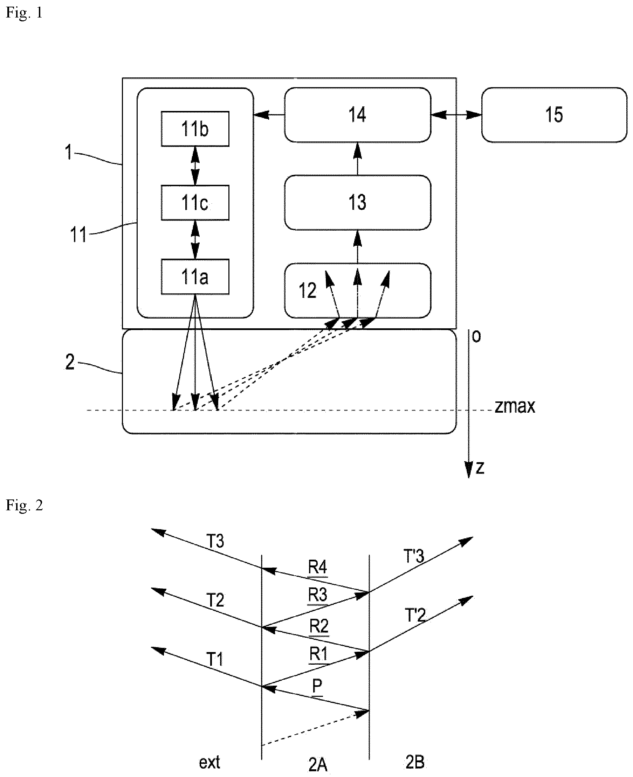

An exemplary non-invasive sensor 1 for implementing the process according to the invention is shown schematically in Figure 1. It comprises:

- an irradiation device 11 comprising a light source 11a, a device for modulating the intensity of this light source 11b, a control device 11c of at least one modulation frequency at which the intensity modulation device 11b modulates the intensity of a light emitted by the light source 11a;
- at least one detection cell 12 configured for detecting a signal generated in response to an irradiation of a target multilayer medium 2 with the irradiation device 11, for example for detecting a thermal wave that propagates in a target multilayer medium 2;
- a signal processing module 13 configured to receive and process data from the at least one detection cell 12;
- an adaptation module for adapting 14 the irradiation parameters of the irradiation device 11;
- optionally, a simulation module 15.

All the elements of the non-invasive sensor 1 can be embedded in the same housing or part of these elements can be remote, in particular the simulation module 15 and/or the adaptation module 14, so that the non-invasive sensor 1 forms a distributed environment.

In the case where parts of the elements of the non-invasive sensor 1 are remote, the non-invasive sensor 1 comprises means of communication so that to allow the required data exchanges.

**Light source 11a**

In a particular embodiment, the light source 11a emits an intensity modulated laser beam at at least one particular wavelength $\lambda$ towards the target multilayer medium 2.

The at least one wavelength can be chosen according to one of the parameters of interest. For example, the wavenumber 1034 cm$^{-1}$, corresponding to a maximum of absorption of glucose, may be relevant if the non-invasive sensor 1 is a blood glucose sensor.

The light source 11a can be a light-emitting diode (LED) or a laser device. The light source 11a can, in addition or in variant, include a quantum cascade laser (QCL) emitting in the mid-infrared region (MIR-QCL), an ICL laser ("Interband Cavity Laser"), a laser having an internal or external cavity, a GaSb laser. These examples are not limiting. The light source 11a can be chosen according to the target multilayer medium 2 and / or parameters of interest.

The non-invasive sensor 1 can include several different and/or identical 11a light sources.

The non-invasive sensor 1 also includes the circuitry associated with the light source(s) 11a and at least one 11c control device configured to control the frequency at which at least one intensity modulation device 11b modulates the intensity of at least one light source 11a, so that the intensity of the light emitted by the light source 11a is modulated at an adjustable modulation frequency.

A modulation frequency of the intensity of the light emitted by a given light source 11a at a particular wavelength $\lambda$ is called hereafter fmod. Several modulation frequencies can be associated with the same wavelength and several wavelengths can be associated with (or equivalently correspond to) the same modulation frequency such that the light emitted by a given light source 11a can be described in terms of pairs, each pair comprising a wavelength 1 and a corresponding modulation frequency fmod.

The light source 11a can be intensity-modulated by means of any known electrical or mechanical means.

The light irradiating the target multilayer medium 2 can be emitted continuously or pulsed by a given light source 11a.

The incident light on the target multilayer medium 2, emitted by the intensity modulated light source(s) 11a, propagates towards the target multilayer medium 2 and then through this target multilayer medium 2 (phenomenon symbolized by solid lines arrows on Figure 1). It is then gradually absorbed by the different constituents of this target multilayer medium 2, up to a characteristic depth called zmax which depends on the structure of the target multilayer medium 2 and its physico-chemical composition.

The absorption of light energy causes local heating of the target multilayer medium 2. As a result, a thermal wave having a frequency equal to the modulation frequency fmod of the light source propagates in the target multilayer medium 2 (phenomenon symbolized by dotted lines arrows on Figure 1), in particular towards the surface of the target multilayer medium 2.

This thermal wave gives rise in the fluid medium surrounding the target multilayer medium 2 to a pressure wave having the same frequency (that is to say a frequency equal to the modulation frequency fmod), which propagates in this fluid medium, for example gaseous and in particular in the detection cell 12 (phenomenon symbolized by alternating dotted arrows in Figure 1). At this stage, the interference phenomenon is not described.

**Detection cell 12**

The detection cell 12 comprises, in the case of photoacoustic detection, a chamber filled with a gas (e.g., air) through which the acoustic wave propagates.

The detection cell 12 also comprises one or more suitable sensors placed in this chamber, for example facing the target multilayer medium 2. For example, the detection cell 12 can comprise one or more electroacoustic sensors configured to convert the pressure of the acoustic wave into an electrical signal, such as a microphone or a piezoelectric transducer.

Each electroacoustic sensor is functionally connected to a signal processing module 13.

**Signal processing module 13**

The signal processing module 13 can comprise an analog-to-digital converter configured to convert an analog electrical signal received from an electroacoustic sensor into a digital signal. The signal processing module 13 can comprise a synchronous sensing device configured for demodulating and extracting the signal of interest from the detected signal.

The signal processing module 13 optionally comprises an operational amplifier operationally connected to the analog-to-digital converter and configured to amplify an electronic signal derived from an acoustic response of the target multilayer medium 2 sensed and transmitted by an electroacoustic sensor.

In particular, the signal processing module 13 is configured for determining an amplitude and a phase for each pair of wavelength and associated modulation frequency of the signal detected by the detection cell 12.

In examples of embodiments, the analog-to-digital converter is functionally connected to a digital signal processor for digital signal processing.

The non-invasive sensor 1 according to the invention further comprises an adaptation module 14 for adapting the irradiation parameters.

**Adaptation module 14**

The adaptation module 14 is a computerized device comprising at least one processor:

- which can receive data from the detection cell 12 and/or from the signal processing module 13 if necessary;
- which can transmit information to the control device(s) 11c of at least one irradiation parameter of the light source 11a (for example a modulation frequency at which the intensity modulation device 11b modulates the intensity of a light emitted by the light source 11a, a wavenumber of a light emitted by the light source 11a, a power of the light source 11a, etc.);
- and if applying, which can exchange data with the simulation module 15 described below.

The adaptation module 14 further comprises at least one memory for storing :

- an abacus of correlation cases which will be described below,
- and optionally a physiological database.

This storage memory can be distributed and/or within the same housing as the other parts of the non-invasive sensor 1.

The adaptation module 14 is configured for carrying out the steps of the method which make it possible to choose the irradiation parameters for the measurement on the basis of the abacus of correlation cases and to determine the sense of variation of the parameter of interest with an amplitude and/or a phase of a wave detected by the detection cell 12. In particular, the signal processing module 13 exchanges data with the adaptation module 14, in particular data associated with one or more amplitude and/or one or more phase of the detected signal.

The database of model configurations and the abacus of correlation cases can be obtained based on real experiments and/or in silico experiments. In a particular embodiment, the non- invasive sensor 1 comprises a simulation module 15 configured to perform in silico experiments. Such a simulation module 15 will be described later in relation with the thermal wave interference phenomenon.

**Thermal wave interference phenomenon**

Since the target multilayer medium 2 comprises at least two superimposed layers with different physico-chemical characteristics, it has at least one interface at which the incident wave can be reflected.

In a simple case, shown in Figure 2, in which the target multilayer medium 2 comprises two layers 2A and 2B, the upper layer 2A is in contact with the outer medium at the 2A/ext interface and with the lower layer at the 2A/2B interface.

The upper layer 2A is for example the stratum corneum of the skin of a patient and the lower layer 2B is for example the epidermis of the skin of a patient in the case of a non-invasive sensor 1 suitable for a measurement of blood glucose.

Even in such a simplified case, the extensive description of the thermal wave generated in the target laminate medium 2 requires the use of complex models, such as the so-called "Hu model", described in Hu, Hanping et al. "Generalized theory of the photoacoustic effect in a multilayer material." Journal of Applied Physics 86 (1999): 3953-3958. This model implicitly includes some of the effects of thermal interference but does not take into account interference due to multiple reflections on interfaces. Other models propose to consider these multiple reflections, including the one described by Cao, J. (2000); Interferential formulization and interpretation of the photoacoustic effect in multi-layered cells ; Journal of Physics D: Applied Physics, 33(3), 200.

Whichever model is chosen, the interference phenomenon has a significant impact on the resulting wave that is further detected. This impact depends on the structuring of the target laminated medium 2.

Figure 2 - although having only pedagogical value - helps understand this impact in a rough way. In the case of figure 2, a so-called "primary" thermal wave P is generated by the irradiation (symbolized by the dotted arrow) at the 2A/2B interface. This primary wave P propagates in particular in the direction of the interface 2A/ext, on which it is partially reflected, which gives rise to a first transmitted wave T1 transmitted in the external medium, and a first reflected wave R1. The first reflected wave R1 can itself reflect on the 2A/2B interface to give rise to a first transmitted wave T'2 transmitted in the second layer 2B and a second reflected wave R2. The second reflected wave R2 can itself give rise, after propagation to the interface 2A/ext, to a second transmitted wave T2 transmitted in the external medium and a third reflected wave R3, and so on.

All the waves transmitted in the external medium (T1, T2, etc.), can interfere with each other so that the amplitude and the phase of the resulting wave that forms in the external environment in response to irradiation are affected. In the exemplary case of figure 2, the amplitude of the thermal wave at the 2A/air interface has the following expression:

Formula 1:

$$\theta = T_{2A/ext} R_{2A/2B} \frac{1}{1 - R_{2A/2B} R_{2A/ext} \, exp\left(-2(1+j)l_2 \sqrt{\frac{\omega}{2\alpha_2}}\right)}$$

where:

- $R_{2A/2B} = \frac{\varepsilon_{2A} - \varepsilon_{2B}}{\varepsilon_{2A} + \varepsilon_{2B}}$ is the coefficient of thermal reflection at interface 2A/2B;

- $R_{2A/ext} = \frac{\varepsilon_{2A} - \varepsilon_{ext}}{\varepsilon_{2A} + \varepsilon_{ext}}$ is the coefficient of thermal reflection at the interface 2A/ext;

- $T_{2A/ext} = \frac{2\varepsilon_{ext}}{\varepsilon_{2A} + \varepsilon_{ext}}$ is the coefficient of thermal transmission at the interface 2A/ext;

- $\alpha_2$ is the thermal diffusivity of the medium 2A;

- $l_2$ is the thickness of layer 2A
- $\varepsilon_{2A}$, $\varepsilon_{2B}$, $\varepsilon_{ext}$ are the respective thermal effusivities of layer 2A, layer 2B and the outer medium.

The thermal effusivities as well as the thickness of layer 2A can, in the case of our target multilayer medium 2, vary over time. In particular, where the target multilayer medium 2 is the skin and the non-invasive sensor 1 is a blood glucose sensor, the thickness of the skin layers, their water concentrations and glucose concentrations, which are likely to affect the different thermal effusivities, evolve over time, so that at the same detection site, the photoacoustic or photothermal signal is affected.

Figure 3 shows the impact on the interference phenomenon on the amplitude (upper graph) and the phase (lower graph) of the resulting thermal wave in the case of a skin model comprising two layers :

- an upper layer modelling the stratum corneum, this upper layer having a thickness of 18 $\mu$m (micrometers) and a water content of 20% and no glucose;
- and a lower layer modelling the epidermis, having a water content of 60%, a glucose content equal respectively 50 mg/dL ; 137.50 mg/dL ; 225 mg/dL ; 312.5 mg/dL and 400 mg/dL (depending on the curve observed) and having an infinite thickness.

These curves have been obtained with the simulation module 15 developed by the inventors and described in European patent application EP22173132.

Such a simulation module 15 allows, among other things, to simulate the photothermal signal associated with an irradiation the characteristics (power, wavelength(es), modulation frequency(ies) ...) of which are known, the target multilayer medium 2 being also modeled by a multilayer medium the characteristics (thicknesses, compositions...) of which are known, on the basis of a multiphysic analytical model taking into account at least implicitly the phenomenon of thermal interference.

**Target multilayer medium 2 model configurations**

In general, the target multilayer medium 2 separates a so-called external medium M_ext from a so-called internal medium M_int and consists of or can be modeled by a succession of N layers. One can for example assume that the interfaces between two successive layers are locally planar.

A model configuration CMk (k being a positive integer) of the target stratified medium 2 corresponds to (or equivalently models) a particular state of the target stratified medium 2. For each model configuration CMk, each layer i ( $i \in [\![1,N]\!]$ ) is described a certain number of explicit parameters (called level 1 parameters because their values will be provided as input to the simulation module 15 for each simulation (or equivalently each in silico experiment), if applying) considered to be suitable for modeling the target stratified medium 2, the parameter(s) of interest not being level 1 parameters.

One considers that a model configuration CMk comprising a number of layers N and a set of level 1 parameters in association with a corresponding set of parameters of interest allows to model a particular state of the target stratified medium 2.

By way of example, if the target multilayer medium 2 is the skin and one intends to measure a glucose concentration in the layer j of this target multilayer medium 2, the target multilayer medium 2 can be considered as entirely described by:

- a model configuration comprising its number of layers N, and for each layer i, its thickness e_i, and its water concentration [H$_2$O]_i,
- and the concentration [Glc]_j of interest.

It is also possible to take into account the concentrations of glucose [Glc]_i for i≠j. In this case the values of e_i ( $i \in [\![1,N]\!]$ ), the concentrations [H$_2$O]_i ( $i \in [\![1,N]\!]$ ) and the concentrations [Glc]_i for ( $i \in [\![1,N]\!]$, $i \neq j$, i ≠ j) are level 1 parameters.

The list of level 1 parameters can be enriched if one wishes to carry out a more precise modeling. In particular, the concentrations of other components of the skin such as lipids, lactate, oxygen, etc., can be included in the list of level 1 parameters describing a layer of the skin.

Still for the example of the skin, it is possible to consider taking into account the color of the skin, the age of the patient, or any other anthropometric parameter, so as to widen or restrict the space of possible models. These are non-limiting examples.

A set of model configurations can be prepared on the basis of real experiments and/or in silico experiments, among others performed with the simulation module 15.

**Simulation module 15**

The simulation module 15 comprises data processing means configured to generate:

- a set of model configurations CMk of the target multilayer medium 2, each of them corresponding (or equivalently modeling or describing) to a particular state of the target stratified medium 2,
- a set of irradiation cases Cirrad (each of them comprising at least one parameter irradiation such as a light power, a wavelength and/or a modulation frequency) of the target multilayer medium 2,
- and the photoacoustic (or photothermal) signals theoretically detected in response to each case of irradiation for each model configuration CMk of the target multilayer medium 2 associated with any particular set of parameters of interest, based on analytical models of the target multilayer medium 2 and of the photoacoustic (or photothermal) detection cell 12.

This simulation module 15 is particularly relevant when the target multilayer medium 2 can evolve over time. In this case, the target multilayer medium 2 indeed adopts different real configurations over time (one or more concentrations vary within one or more layers of the target multilayer medium 2; one or more dimensions of the target multilayer medium 2 can vary such as, for example, the thickness of one of the layers). Each real configuration can be modeled by a particular model configuration CMk.

A multiphysic analytical model of the target multilayer model 2 is for example based on physical and/or chemical equations such as, by way of non-limiting example, the Beer-Lambert equations for optical absorption and the equations thermodynamics of heat (Fourier's law and conservation laws).

The model partly described in the present application were developed in the case of skin but the person skilled in the art will adapt it without difficulty to another type of medium merely by selecting suitable types and/or ranges of level 1 parameters and/or suitable physical or chemical equations for the target multilayer medium 2 of interest.

For each model configuration CMk, the multiphysic analytical model makes it possible, if the parameter(s) of interest is (are) also known, to simulate the thermal wave generated at the interface layer 1/external medium M_ext (interface 1/M_ext) in response to an irradiation by a light source 11a whose irradiation parameters are known, namely for example at least one modulation frequency fmod, at least one respective associated wavelength $\lambda$ and at least one respective surface power density.

As a variant, the multiphysic analytical model makes it possible to simulate the pressure wave generated in the external medium M_ext.

In both cases, the signal obtained at the output of a processor implementing the multiphysic analytical model is called "simulated response wave".

The simulated response wave can be provided as input to a processor implementing a detection cell model. For example, such a processor is configured to generate a theoretical output signal of the detection cell 12 when this detection cell 12 receives as input a simulated response wave.

The simulation module 15 made it possible to carry out extensive in silico experiments, in particular in the case of a glycemia sensor, part of the results of which are presented on figure 3. In the case of Figure 3a, one simulates the irradiation of a skin model configuration with a laser light having a wavenumber of 1034 cm$^{-1}$ and intensity-modulated at a modulation frequency fmod that is plotted on the x-axis.

The model configuration CMk for figure 3a consists of two layers (N =2):

- an upper layer L1 modelling the stratum corneum, this upper layer having a thickness of 18 $\mu$m (micrometers), a water content of 20% and no glucose (level 1 parameters : e_L1=18 $\mu$m, percentage_H2O_L1 =20 % , [Glc]_L1 = 0);
- and a lower layer L2 modelling the epidermis, having a water content of 60% (level 1 parameters : e_L2= $\infty$, percentage_H2O_L2 =60 %).

The amplitude of the thermal simulated response wave generated in response to this irradiation is plotted on the y-axis as a function of the modulation frequency fmod for different lower layer blood glucose values (50.0 mg/dL ; 162.5 mg/dL ; 275.0 mg/dL and 387.5 mg/dL), the lower layer blood glucose ([Glc]_2) value being the parameter of interest.

Unexpectedly, the inventors observed the existence of so-called "nodal" modulation frequencies near which the amplitude and/or the phase of the resulting thermal wave is independent of the parameter of interest, in this case blood glucose.

In the case of figure 3a,

- two nodal modulation frequencies can be observed concerning the upper graph, namely fnod1_amp = 530 Hz and fnod2_amp = 3 000 Hz,

- two nodal modulation frequencies can be observed concerning the lower graph, namely fnod1_ph = 55 Hz, fnod2_ph = 1 440 Hz,

but there are still others nodal modulation frequencies outside the modulation frequency range shown in figure 3a. These nodal modulation frequencies can be explained by the phenomenon of thermal wave interference within the target multilayer medium 2.

In the case of Figure 3c, one simulates the irradiation of a skin model configuration with a laser light having a wavenumber of 1125 cm$^{-1}$ and intensity-modulated at a modulation frequency fmod that is plotted on the x-axis.

The model configuration CMk for figure 3c consists of two layers (N =2):

- an upper layer L1 modelling the stratum corneum, this upper layer having a thickness of 18 $\mu$m (micrometers), a water content of 20% and no lactate (level 1 parameters : e_L1=18 $\mu$m, percentage_H2O_L1 =20 %, [lactate]_L1 = 0);
- and a lower layer L2 modelling the epidermis, having a water content of 60% (level 1 parameters : e_L2= $\infty$, percentage_H2O_L2 =60 %).

The amplitude of the thermal simulated response wave generated in response to this irradiation is plotted on the y-axis as a function of the modulation frequency fmod for different lower layer blood lactate values (30.0 mg/dL ; 72.5 mg/dL ; 115.0 mg/dL and 157.5 mg/dL), the lower layer blood lactate value ([lactate]_2) being the parameter of interest.

As well as in the case of figure 3a, nodal frequencies are observed on figure 3c. In the case of figure 3c,

- one nodal modulation frequency can be observed concerning the upper graph, namely fnod1_amp = 450 Hz,
- one nodal modulation frequency can be observed concerning the lower graph, namely fnod1_ph = 42 Hz,

but there are still others nodal modulation frequencies outside the modulation frequency range shown in figure 3c.

Anyway, figure 3a and figure 3c show that the principle of the nodal modulation frequencies isn't linked to a particular parameter of interest and hence, that the adaptation of the process according to the invention requires only to adapt the irradiation configuration and the level 1 parameters of the model configuration CMk to the case in question.

Each nodal modulation frequency corresponds to a response wave generated in the medium whose amplitude is weakly correlated with the parameter of interest, or even independent of the parameter of interest (in this case: interstitial glycemia), so the signal detected at such a modulation frequency cannot be used for the determination of this parameter of interest.

In general, the extensive in silico experiments carried out by the inventors have therefore enabled them to observe the following facts:

- nodal modulation frequencies exist, near which the amplitude or the phase of the thermal wave generated in response to irradiation is weakly correlated with the parameter of interest, or even independent of the parameter of interest. It will be noted that if we enlarge the scale of figure 3a around fnod1_amp as represented on figure 3b, a nodal frequency is not really a single nodal modulation frequency but a limited frequency range in which the thermal amplitude varies much less than the parameter of interest varies in terms of relative variations. More specifically, in the case of figures 3a and 3b, the range for fnod1_amp is [500, 535 Hz] corresponding to a relative uncertainty of only 7%.
- the particular values of the nodal modulation frequencies depend on the characteristics of the target stratified medium 2, namely in particular the thicknesses and compositions of the different layers of the target stratified medium 2;
- the values of the nodal modulation frequencies for the amplitude fnod_amp are different from the values of the nodal modulation frequencies for the phase fnod_ph of the resulting wave (and in general of the detected wave), which give rise to four different correlation cases CC. A correlation case is a triplet of three signs :
- the sign sgn(Rparam/amp) of the correlation coefficient Rparam/amp between the amplitude of the detected wave and the parameter to be measured;
- the sign sgn(Rparam/ph) of the correlation coefficient Rparam/ph between the phase of the detected wave and the parameter to be measured;
- the sign sgn(Rph/amp) of the correlation coefficient Rph/amp between the amplitude and the phase of the detected wave.

In other words, a correlation case CorrCase is a triplet that can be noted (sgn(Rparam/amp); sgn(Rparam/ph); sgn(Rph/amp)). Of course, the three elements of a correlation case are note independent from each other but any two elements of a correlation case are independent. Hence, there are four different correlation cases:

- correlation case 1 : CorrCase_1 = ( + ; - ; -), corresponding to zones 1 on figures 3 to 5 ;

- correlation case 2 : CorrCase_2 = ( - ; - ; +), corresponding to zones 2 on figures 3 to 5 ;
- correlation case 3 : CorrCase_3 = ( - ; + ; -), corresponding to zones 3 on figures 3 to 5 ;
- correlation case 4 : CorrCase_4 = ( + ; + ; +), corresponding to zones 4 on figures 3 to 5.

It is very difficult to make enough in vivo observations to obtain such curves. These observations were therefore only made possible thanks to the simulation module 15 developed by the inventors.

Thanks to this simulation module 15, the inventors have been able to understand and exploit all the aspects of this interference phenomenon.

The inventors have performed further simulations to understand how the nodal modulation frequencies, and hence the correlation case associated with a given modulation frequency, evolve with the structure of the target multilayer medium 2, or in other words with the level 1 parameters and the parameters of interest of the model configuration CMk for the target multilayer medium 2.

In particular, the inventors have performed the same simulations as for figure 3 but with varying level 1 parameters.

The results can be observed on:

a- figure 4a which represents the two first nodal modulation frequencies for the amplitude of the thermal wave fnod _amp_1 and fnod_amp_2 and the two first nodal modulation frequencies for the phase of the thermal wave fnod_ph_1 and fnod_ph_2 as a function of the thickness of the upper layer in the case of a skin model comprising two layers:

- an upper layer modelling the stratum corneum, this upper layer having a thickness varying between 10 $\mu$m and 40 $\mu$m, a water content of 20% and no glucose;
- and a lower layer modelling the epidermis, having a water content of 60% and having an infinite thickness.

One can see that in this particular case, fnod _amp_1, fnod_amp_2, fnod_ph_1 and fnod_ph_2 decrease when the thickness of the upper layer increases, all other things being equal.

b- figure 4b which represents the two first nodal modulation frequencies for the amplitude of the thermal wave fnod _amp_1 and fnod_amp_2 and the two first nodal modulation frequencies for the phase of the thermal wave fnod_ph_1 and fnod_ph_2 as a function of the water content of the upper layer in the case of a skin model comprising two layers:

- an upper layer modelling the stratum corneum, this upper layer having a thickness of 18 $\mu$m, a water content varying from 15 % to 30% and no glucose;
- and a lower layer modelling the epidermis, having a water content of 60% and having an infinite thickness.

One can see that in this particular case, fnod _amp_1, fnod_amp_2, fnod_ph_1 and fnod_ph_2 slightly decrease when the water content of the upper layer increases, all other things being equal.

c- figure 4c which represents the two first nodal modulation frequencies for the amplitude of the thermal wave fnod _amp_1 and fnod_amp_2 and the two first nodal modulation frequencies for the phase of the thermal wave fnod_ph_1 and fnod_ph_2 as a function of the water content of the lower layer in the case of a skin model comprising two layers:

- an upper layer modelling the stratum corneum, this upper layer having a thickness of 18 $\mu$m, a water content of 20% and no glucose;
- and a lower layer modelling the epidermis, having a water content varying from 40% to 80% and having an infinite thickness. One can see that in this particular case, fnod_amp_1,

fnod_amp_2, fnod_ph_1 and fnod_ph_2 slightly increase when the water content of the lower layer increases, all other things being equal.

Of course, it is possible to vary all of the level 1 parameters of the model configuration CMk for the target multilayer medium 2, a representation in two dimensions of all the possible cases being impossible.

In general, one understands that at a given modulation frequency and a given wavelength of the irradiating light, a given structure of the target multilayer model 2 is associated with a given correlation case CC among the four possible correlation cases.

As a consequence, the three following items :

- the irradiation case Cirrad, characterized by at least one wavelength $\lambda$ of the irradiating light, and for each of these wavelengths, at least one respective power P and at least one respective modulation frequency fmodof the irradiating light ;
- the level 1 parameters of the model configuration CMk of the target multilayer medium 2, which is to say all parameters including:

   * a number N of layers,
   * for each layer i : a thickness e_i and at least one numeric value X_i characterizing its chemical composition, X_i being different from the parameter(s) of interest;

- the three signs of the correlation case

are not independent from each other, so that the knowledge of part of these three items can help determine part of all or the complementary unknown part of these three items.

**Abacus of correlation cases**

In a particular embodiment, the first step of the process consists in obtaining an abacus of correlation cases comprising for a plurality of pairs (CMk, Cirrad), the corresponding correlation case CorrCase.

The abacus of correlation cases can be obtained by means of real experiments and/or in silico experiments, for example with the simulation module 15.

Figure 5 shows part of an abacus of correlation cases obtained with a simulation module 15 with:

- a set of irradiation configurations Cirrad comprising a wavenumber v of 1034 cm$^{-1}$ and a modulation frequency fmod ranging from 50 Hz to 4000 Hz
- and a set of model configuration CMk comprising {N = 2 layers, layer 1 (upper layer) : thickness e_L1= 18 $\mu$m or 35 $\mu$m; water percentage perc_H2O_L1 = 0%; [Glc]_L1 = 0 , layer 2 : thickness e_L2 = infinite ; water percentage perc_H2O_L2 ranging from 40% to 80 %; [Glc]_L2 = 110 mg/dL)}.

Note that since the nodal frequencies are by definition not (or very little) dependent on the parameter of interest, the particular value chosen for [Glc]L2 has very little impact on these abacus of correlation cases.

Figures 4a, 4b and 4c show clearly that the most significant level 1 parameter is the upper layer thickness and that in the typical physiological ranges accessible for the water content of this upper layer (modelling the stratum corneum) and for the water content of the lower layer (modelling the epidermis), these two level 1 parameters don't influence significantly the correlation case associated with each modulation frequency in the range ]0 Hz, 3000 Hz]. Since the water content of the upper layer modeling the stratum corneum has been observed to only slightly influence the correlation case associated with a given modulation frequency, the abacus of figure 5 that sets the water content of the upper layer modeling the stratum corneum to 20% could be sufficient in itself to implement the process according to the invention in the case of non-invasive glycemia sensor 1.

Preferably, the set of model configurations CMk implemented in the abacus of correlation cases is representative of the real sample space for the target multilayer medium 2.

Depending on the target multilayer medium 2, the cardinal of the plurality of sets of model configurations CMk of the abacus of correlation cases can be limited or reduced, based on the dependencies of the nodal modulation frequencies on the level 1 parameters, on the principles described above in the case of figure 5 or in the case of Table 1 and Table 2 below.

For example, in the case of figures 4a, 4b and 4c, it appears that the senses of variation of fnod_amp_1, fnod_amp_2, fnod_ph_1 and fnod_ph_2 as a function of the upper layer thickness (respectively the upper layer water content, respectively the lower layer content) are constant. These observations allow to define two limiting model configurations CM1 and CM2 and two associated limiting sets of level 1 parameters:
The set of level 1 parameters of the first limiting model configuration CM1 is the following one: CM1 ={2 layers, layer 1 (upper layer) : thickness e_L1= 8 $\mu$m ; water percentage perc_H2O_L1 = 0%; [Glc]_L1 =0 , layer 2 : thickness e_L2= infinite ; water percentage perc_H2O_L2 = 75 %)}

The set of level 1 parameters of the second limiting model configuration CM2 is the following one:
CM2 ={2 layers, layer 1 (upper layer) : thickness e_L1= 35 $\mu$m ; water percentage perc_H2O_L1 = 85 %, layer 2 : thickness e_L2= infinite ; water percentage perc_H2O_L2 = 50 %)}.

The correlation case has been determined by means of simulation for each of the limiting cases at different modulation frequencies fmod ranging from 100 Hz to 1500 Hz, the wavenumber of the irradiation light being in each case equal to 1034 cm$^{-1}$. The results of these simulations are reported :
- in Table 1 below in the case of CM1:

**Table 1 : Correlation Case as a function of fmod in the case of model configuration CM1**

| fmod (Hz) | sgn(Rparam/amp) | sgn(Rparam/ph) | sgn(Rph/amp) | CorrCase |
|---|---|---|---|---|
| 100 | + | + | + | 4 |
| 200 | + | + | + | 4 |
| 300 | + | + | + | 4 |
| 500 | + | + | + | 4 |
| 600 | + | + | + | 4 |
| 800 | + | + | + | 4 |
| 1000 | + | + | + | 4 |
| 1500 | + | + | + | 4 |

- and in Table 2 below in the case of CM2 :

**Table 2 : Correlation Case as a function of fmod in the case of model configuration CM2**

| fmod (Hz) | sgn(Rparam/amp) | sgn(Rparam/ph) | sgn(Rph/amp) | CorrCase |
|---|---|---|---|---|
| 100 | + | - | - | 1 |
| 200 | - | - | + | 2 |
| 300 | - | - | + | 2 |
| 500 | - | + | - | 3 |
| 600 | - | + | - | 3 |
| 800 | - | + | - | 3 |
| 1000 | + | + | + | 4 |
| 1500 | + | - | - | 1 |

Several tables of correlation cases such as Table 1 and Table 2 described below, or more generally an abacus of correlation cases such as the one represented on figure 5 can be stored in the storage memory of the non-invasive sensor 1.

In the example of Table 1 and Table 2, based on the two limiting cases CM1 and CM2, one understands that with the particular irradiation case Cirrad_part characterized by a wavenumber of 1034 $cm^{-1}$ and a modulation frequency fmod = 100 Hz, only two correlation cases can occur in the physiological ranges admissible for the level 1 parameters and the parameter of interest which is in this case the glucose content of the lower layer modeling the epidermis.

As a consequence, if the correlation coefficient Rph/amp between the amplitude and the phase of the thermal or pressure wave generated in response to an irradiation is calculated with the signal processing module 13 based on the signals detected by the detection cell 12 in response to a plurality of successive irradiations with the irradiation configuration Cirrad_part,

- either the sign sgn(Rph/amp) is positive, which means that the target multilayer medium is in correlation case 4 and that the parameter of interest increases when the amplitude of the wave detected by the detection cell 12 increases and when the phase of the wave detected by the detection cell 12 increases;
- or the sign sgn(Rph/amp) is negative, which means that the target multilayer medium is in correlation case 1 and that the parameter of interest increases when the amplitude of the wave detected by the detection cell 12 increases and when the phase of the wave detected by the detection cell 12 decreases.

As a consequence, if the tables of correlation cases for the two limiting model configurations CM1 and CM2, or more generally for a representative set of model configurations are known and stored in a storage memory of the non-invasive sensor 1, it is possible to determine the sense of variation of the parameter of interest without preliminary measurements with additional invasive and/or non-invasive sensors to determine the structure of the target multilayer medium 2, and without determining the value of the parameter of interest at different time points.

Hence, the method for determining the sense of variation of at least one parameter of interest in a target multilayer medium 2 according to the invention can comprise, as represented on the flowchart of figure 6:

- 61 : choosing a model configuration CMk_part of the target multilayer medium 2 among a representative set of model configurations, that is to say a set of model configurations that is representative of the real space of possible level 1 parameters for the particular target multilayer medium 2 to be analyzed.
- 62 : choosing for subsequent irradiation an irradiation case Cirrad(CMk_part) consistent with the model configuration CMk_part on the basis of an abacus of correlation cases.
- 63 : performing a plurality of successive irradiations of the target multilayer medium 2 with the light source 11a of the non-invasive sensor 1 configured with the chosen irradiation configuration Cirrad(CMk_part), detecting with the detection cell 12 each of the waves generated in response to these irradiations and determining with the signal processing module 13 their respective amplitude and phase at at least one wavelength λ and at least one respective associated modulation frequency fmod of the determined irradiation case Cirrad.
- 64 : determining a sign sgn(Rph/amp) of a correlation coefficient Rph/amp between the amplitude and the phase of the detected waves for at least one of the at least one wavelength λ and a corresponding modulation frequency fmod of the chosen irradiation case Cirrad(CMk_part).
- 65 : determining at least one correlation case with the processor of the adaptation module 14 based on the abacus of correlation cases, on the determined sign sgn(Rph/amp(λ, fmod)) for the at least one wavelength λ and the at least one respective associated modulation frequency fmod of the determined irradiation case Cirrad (CMk_part) and on the model configuration CMk_part.
- 66 : determining the sense of variation of at least one parameter of interest based on the at least one correlation case.

On flowcharts 6 to 9, only in the cases where several types of arrows enter a given node symbol, the only possible pathway associated with a particular type of input arrow is to continue with the output arrow of the same type. For example, on flowchart 6, step 64 is followed by step 65 through the abacus of correlation cases and step 61 is followed by step 62 through the abacus of correlation cases, but step 65 can't succeed directly to step 62 through the abacus of correlation cases.

### 61 : Choice of a model configuration CMk_part

Step 61 can be performed by the adaptation module 14 of the non-invasive sensor 1.

Only general information on the target multilayer medium 2 are needed at step 61, that is to say information sufficient to determine a particular model configuration CMk_part.

For example, a particular model configuration CMk_part is a mean model configuration or equivalently the most likely model configuration CMk for the target multilayer medium 2 among the representative set of model configurations.

The likelihood associated with each of the model configuration CMk can be calculated based on previous observations made on different target multilayer media 2 equivalent to the target multilayer medium 2 of interest.

For example, in the case of glycemia, data of a cohort of patients can be used to determine the particular model configuration CMk_part.

In addition or in another embodiment, previous observational data of the individual on which the glycemia sensor is to be implemented can be taken into account to determine the initial model configuration CMk_part. In particular, the particular model configuration CMk_part could depend on the expected physical activity (meal, sleep, etc.) at the time or slightly before the irradiations.

In a particular embodiment, the adaptation module 14 comprises a memory for storing a physiological database. Optionally, the choice of the particular model configuration CMk_part is based at least partly on this physiological database.

### 62 : Choice of an irradiation configuration Cirrad(CMk_part)

Step 62 can be performed by the adaptation module 14 of the non-invasive sensor 1.

The adaptation module 14 receives at step 62 the particular model configuration CMk_part determined at step 61 and determines on the basis of the abacus of correlation cases an irradiation configuration Cirrad(CMk_part) consistent with CMk_part.

An irradiation configuration Cirrad is considered as consistent with a particular model configuration CMk_part if for the whole representative set of model configurations, this irradiation configuration is associated with two or less correlation cases among the four correlation cases possible.

For example, in the case of the set of model configurations explored in figures 4a, 4b, 4b, if one knows that the stratum corneum has mandatorily a thickness comprised between 10 μm and 30 μm, these limits defining the representative set of model configurations in this case (dotted square on figure 4a), one can see that if fmod = 500 Hz, only the correlation cases associated zone 1 and zone 2 can occur (horizontal dotted line on figure 4a). As a consequence, an irradiation configuration Cirrad(CMk_part) comprising a light having a wavenumber v = 1034 cm⁻¹ and a corresponding intensity modulation frequency fmod = 500 Hz would be considered as consistent with any particular model configuration CMk_part

Other choices could be possible, and several additional criteria can be taken into account for choosing Cirrad(CMk_part), among which some of the values of the level 1 parameters of CMk_part.

### 63 : plurality of irradiations and detections

Once the irradiation configuration Cirrad(CMk_part) has been chosen, it is transmitted to the irradiation device 11 so that the light source 11a can be configured in the chosen irradiation configuration Cirrad(CMk_part) to irradiate the target multilayer medium 2.

A plurality of successive irradiations and the respective detections with the detection cell 12 are performed at step 63.

Two successive irradiations can be separated by a fixed time interval or by different time intervals.

The plurality of irradiations can be performed within a predetermined irradiation duration Tirrad. Tirrad can be chosen so as to be less than the characteristic time of the variations of one or more of the level 1 parameters, for example less than 2, 4, 6, 8 or 10 times the smallest characteristic time among all the characteristic times of the variations of the level 1 parameters.

### 64 : determination of sgn(Rph/amp)

At step 64, the signal processing module 13 receives all the signal detected by the detection cell at step 63 and determines, for at least one wavelength λ and one associated modulation frequency fmod of the chosen irradiation configuration CMk_part, the amplitude and the phase of the signal corresponding to each irradiation.

The processor of the adaptation module 14 or the processor of the signal processing module 13 determines the sign sgn(Rph/amp(λ, fmod)) of the correlation coefficient Rph/amp(λ, fmod) at at least one wavelength λ and the corresponding modulation frequency fmod between all the amplitudes and phases determined at the respective wavelength and modulation frequency.

### 65 : determination of the correlation case

Step 65 can be performed by the adaptation module 14 of the non-invasive sensor 1.

The adaptation module 14 receives as input at step 65 the at least one sign sgn(Rph/amp(λ, fmod)), as well as the respective wavelength λ, the respective associated modulation frequency fmod and the particular model configuration CMk_part of the target multilayer 2 and determines as an output the corresponding correlation case based on the abacus of correlation cases.

For example, in the cases of Figures 4a, 4b and 4c, if the irradiations were performed with v=1034 cm⁻¹ and finod= 500 Hz, and sgn(Rph/amp(λ, fmod)) = +, the correlation case determined is CorrCase_2.

### 66 : determination of the sense of variation of the parameter of interest with amplitude and/or phase

Step 66 can be performed by the adaptation module 14 of the non-invasive sensor 1.

The adaptation module 14 receives as input at step 66 the at least one correlation case determined at step 66 and determines as an output the corresponding sense of variation of the parameter of interest either with the amplitude of the detected signal at the respective wavelength λ and the associated respective intensity modulation frequency fmod, or with the phase of the detected signal at the respective wavelength λ and the associated respective intensity modulation frequency fmod.

For example, if the correlation case determined at step 65 is CorrCase_2, one knows that if the amplitude and/or the phase of the signal detected increases with the same irradiation configuration, the parameter of interest decreases.

In a particular embodiment, once the sense of variation of the parameter of interest with amplitude and/or phase has been determined, at least one additional measurement of the amplitude and/or the phase of the wave generated in response to an additional irradiation with the chosen irradiation configuration Cirrad can be performed. The sense of variation of the amplitude and/or the phase of the detected signal is deduced from the additional measurement and from the preceding measurement.

The sense of variation of the parameter of interest over time is determined based on the sense of variation of the amplitude and/or the phase of the detected signal and on the sense of variation of the parameter of interest with the amplitude and/or the phase determined at step 65. For example, if one states that the amplitude of the signal detected by the detection cell 12 increases between t1 and t2 and it has been determined at step 66 that the parameter of interest is inversely correlated with this amplitude, one can assert that the parameter of interest is decreasing without having to calculate the values of the parameter of interest at t1 and t2.

In this case and if the parameter of interest is blood glucose, the user of the non-invasive sensor 1 can be informed of such a sense of variation of the parameter of interest over time at a particular time point. For example, a message such as "glycemia rising" or "glycemia falling" can be prompted on a user interface.

In another embodiment, an alarm can be raised if a decrease (respectively an increase) of glycemia over time is detected and the previously measured glycemia was more than but close to an inferior (respectively less than but close to a superior) threshold.

In these two specific examples (which could be enlarged to other cases than those specific to glycemia), it is to be noted that the method according to the invention allows the non-invasive sensor 1 to provide information on the parameter of interest without effectively measuring two times this parameter of interest.

In other words, the non-invasive sensor 1 acts as a trend sensor for the parameter of interest, which can in particular have a lower power consumption than a traditional sensor 1 for the parameter of interest.

In another particular embodiment, the determined sense of variation of the parameter of interest over time with the method according to the invention can be compared to the sense of variation of the parameter of interest over time with the same or a different non-invasive sensor configured to measure the parameter of interest. This can reinforce the confidence level in these very measures of the parameter of interest.

**Iterative choice of the model configuration CMk_part**

In a particular embodiment represented on figure 7, once the correlation case has been determined at step 65, the adaptation module 14 can chose a new model configuration CMk_part based on the abacus of correlation cases and on the determined correlation case, and optional additional data from a database associated with the target multilayer medium 2.

In the case of a glycemia sensor, such a database could contain physiological data obtained on a cohort of patients and/or on the particular user of the non-invasive sensor 1.

For example, once again in the cases of figures 4a, 4b, and 4c, if the irradiations were performed with $v=1034$ $cm^{-1}$ and the associated fmod= 500 Hz and the correlation case determined at step 65 was CorrCase_2, one knows thanks to the abacus of correlation cases that the thickness of the stratum corneum is comprised between 20 $\mu$m and 30$\mu$m.

The physiological database can comprise data such as maximum thickness variation over 30 minutes = +/- 2 $\mu$m for the stratum corneum - maximum thickness of the stratum corneum : 30 $\mu$m. The physiological database can comprise other information such as stratum corneum thickness can only increase in case of a meal or a particular physical activity.

As a consequence, one knows thanks to the preceding steps of the method according to the invention that the thickness of the stratum corneum will be comprised between 18 $\mu$m and 30$\mu$m during the next thirty minutes, which allows to reduce the set of representative model configurations as represented with the plain square on figure 4a, which could lead with a new step 61 to a new choice of particular irradiation configuration CMk_part comprising $v=1034$ $cm^{-1}$ and the corresponding fmod = 2000 Hz.

The other steps of the method according to the invention can then be repeated at least one time. Such iterations allow to increase the confidence level in the sense of variation determined at step 66.

In some particular cases, where only one correlation case is accessible, step 63 can be bypassed. This would be the case for a particular irradiation configuration CMk_part comprising $v=1034$ $cm^{-1}$ and the corresponding fmod = 100 Hz where only zone 1 is accessible for the representative set of model configurations represented as a dotted square on figure 4a.

**Induced controlled variation of parameter of interest**

In the cases where the initial data regarding the particular target multilayer medium 2 don't allow one to choose a particular model configuration CMk_part, it is possible to exploit once again the abacus of correlation cases in order to allow this first choice. This embodiment is represented on figure 8.

In this case, the process according to the invention can comprise an optional step 81 for inducing a controlled variation of the parameter of interest in the target multilayer medium 2. For example, in the case of a glycemia sensor, the user can ingest a particular amount of sugar, such that one knows that within a few minutes that follow the sugar intake (let's say N minutes), the glycemia will mandatorily increase.

As a consequence, if step 62 is performed to choose for example a default irradiation configuration adapted for such an induction step 81 comprising at least one wavelength $\lambda$ and a corresponding intensity modulation frequency fmod, and if step 63 is then performed within these N minutes, the processor of the adaptation module 14 can then determine at step 84:

a - the sign sgn(Rparam/amp($\lambda$, fmod)) of the correlation coefficient Rparam/amp($\lambda$, fmod) between the amplitude of the detected wave and the parameter of interest;
b - the sign sgn(Rparam/ph($\lambda$, fmod)) of the correlation coefficient Rparam/ph($\lambda$, fmod) between the phase of the detected wave and the parameter to be measured,

in so far as the sense of variation of the parameter of interest over time is known,

The correlation case is consequently entirely determined, and based on the abacus of correlation cases, the processor of the adaptation module 14 can get additional information on the representative set of model configurations, which allows to perform a new step 61, etc.

By way of example, once again in the case of Figure 4a, if the correlation case determined based on the glucose intake is CorrCase_2 and the particular irradiation configuration CMk_part comprised v=1034 cm$^{-1}$ and the associated fmod= 300 Hz, one can deduce from the abacus of correlation cases that the thickness of stratum corneum is comprised between 10$\mu$m and 13$\mu$m, allowing to make a different choice for the next particular irradiation configuration.

In a particular embodiment, step 81 and 84 can be performed iteratively after step 61, the method comprising in this case a decision step 91 for deciding if the knowledge of the target multilayer medium 2 is sufficient for determining a so-called consistent particular irradiation configuration Cirrad(CMk_part), as represented on the flowchart of figure 9.

## LIST OF THE REFERENCE SIGNS

1: Non-invasive sensor based on indirect photoacoustic detection
11: Irradiation device
11a: light source
11b: device for modulating the intensity of the light source 11a
11c: device for controlling the modulation frequency fmod of the intensity of the light source 11a
12: Detection cell
13: Signal processing module
14: Adaptation module
15: Simulation module
2: Target laminate medium
2A: First layer of the target laminated medium
2B: second layer of the target laminate medium

## Claims

1. Process for analyzing at least one parameter of interest in a target multilayer medium (2) with a non-invasive sensor (1) based on photoacoustic or photothermal detection, the process comprising:

   a) providing a non-invasive sensor comprising :

   - a light source (11a), the irradiation configuration (Cirrad) of which is tunable, this irradiation configuration comprising at least one wavelength ($\lambda$) and at least one respective associated intensity modulation frequency (fmod),
   - a detection cell (12) configured for detecting an acoustic or thermal signal,
   - a signal processing module (13),
   - an adaptation module (14) comprising a processor and a memory, **characterised in** the memory storing an abacus of correlation cases, the abacus of correlation cases comprising, for each of a plurality of pairs comprising one of a plurality of model configuration (CMk) of the target multilayer medium (2) and one of a plurality of irradiation configuration (Cirrad) of the light source (11a), at least one associated correlation case which comprises for at least one wavelength of the irradiation configuration and a respective associated modulation frequency (fmod):

       - a first sign which is the sign of the correlation coefficient (Rparam/amp($\lambda$, fmod)) between the amplitude of the detected wave at the respective wavelength and associated modulation frequency and the parameter of interest,
       - a second sign which is the sign of the correlation coefficient (Rparam/ph($\lambda$, fmod)) between the phase of the detected wave at the respective wavelength and associated modulation frequency and the parameter of interest, and

- a third sign which is the sign of the correlation coefficient (Rph/amp($\lambda$, fmod)) between the amplitude and the phase of the detected wave at the respective wavelength and associated modulation frequency; the method further comprising

b) determining a particular model configuration (CMk_part) of the target multilayer medium (2);

c) determining with the adaptation module (14) a particular irradiation configuration (Cirrad(CMk_part)) for subsequent irradiation based on the abacus of correlation cases and on the particular model configuration;

d) performing a plurality of irradiations of the target multilayer medium (2) during an irradiation time interval (Tirrad) with the light source (11a) configured with the particular irradiation configuration (Cirrad(CMk_part)) determined at step c) and detecting the respective signal generated in response to the irradiation with the detection cell (12) and determining a respective amplitude and a respective phase of said signal at at least one wavelength and at least one respective associated modulation frequency of the particular irradiation configuration with the signal processing module (13);

e) with the adaptation module (14), calculating the third sign of a correlation case associated with at least one of the at least one wavelength and at least one respective associated modulation frequency of the particular irradiation configuration (Cirrad(CMk_part)) based on the determined respective amplitudes and phases;

f) determining with the adaptation module (14) a correlation case associated with at least one of the at least one wavelength and the respective modulation frequency of the particular irradiation configuration (Cirrad(CMk_part)) based on the abacus of correlation cases and the respective calculated third sign;

g) determining with the adaptation module (14) the sense of variation of at least one parameter of interest with an amplitude and/or a phase of the thermal or acoustic signal based on the at least one associated correlation case determined at f).

2. Process for analyzing at least one parameter of interest in a target multilayer medium (2) according to claim 1 comprising after g) : determining the sense of variation over time of at least one parameter of interest based on the signal generated in response to an additional irradiation of the target multilayer medium (2) with the particular irradiation configuration and the sense of variation with the amplitude and/or the phase of the thermal or acoustic signal determined at g).

3. Process for analyzing at least one parameter of interest in a target multilayer medium (2) according to claim 1 or claim 2 comprising after f) and before g) at least one iteration of a), b), c), d) , e) and f);

4. Process for analyzing at least one parameter of interest in a target multilayer medium (2) according to any of claims 1 to 3 comprising before a) :

i- performing an induced controlled variation of the parameter of interest,
ii- performing an additional step c),
iii- performing an additional step e),
iv- with the adaptation module (14), calculating a first sign and a second sign of a correlation case associated with at least one of the at least one wavelength and at least one respective associated modulation frequency of the particular irradiation configuration (Cirrad(CMk_part)) determined at step ii) based on the respective amplitudes and phases determined at step iii),
v- performing an additional step f),

and providing as additional input for a) the correlation case determined at v.

5. Non-invasive sensor (1) for analyzing at least one parameter of interest in a target multilayer medium (2) based on photoacoustic or photothermal detection comprising:

- a light source (11a), the irradiation configuration (Cirrad) of which is tunable, this irradiation configuration comprising at least one wavelength ($\lambda$) and at least one respective intensity modulation frequency (fmod);
- a detection cell (12) configured for detecting an acoustic or thermal signal;
- a signal processing module (13);
- an adaptation module (14) comprising :

1) a processor,

2) a memory; **characterised in** the memory comprising an abascus of correlation cases, the abascus of correlation cases comprising, for each of a plurality of pairs comprising one of a plurality of model configuration (CMk) of the target multilayer medium (2) and one of a plurality of irradiation configuration (Cirrad) of the light source (11a), at least one associated correlation case which comprises for at least one wavelength ($\lambda$) of the irradiation configuration and a respective associated modulation frequency (fmod):

- a first sign which is the sign of the correlation coefficient (Rparam/amp($\lambda$, fmod)) between the amplitude of the detected wave at the respective wavelength and associated modulation frequency and the parameter of interest,
- a second sign which is the sign of the correlation coefficient (Rparam/ph($\lambda$, fmod)) between the phase of the detected wave at the respective wavelength and associated modulation frequency and the parameter of interest, and
- a third sign which is the sign of the correlation coefficient (Rph/amp($\lambda$, fmod)) between the amplitude and the phase of the detected wave at the respective wavelength and associated modulation frequency

the adaptation module being configured for:

* determining a particular model configuration (CMk_part) of the target multilayer medium (2),
*determining a particular irradiation configuration (Cirrad(CMk_part)) for subsequent irradiation based on the abacus of correlation cases and on the particular model configuration,
*calculating a third sign of a correlation case associated with at least one of the at least one wavelength and a respective associated modulation frequency of the particular irradiation configuration (Cirrad(CMk_part)) based on the determined respective amplitudes and phases,
*determining a correlation case associated with at least one of the at least one wavelength and a respective associated modulation frequency of a particular irradiation configuration (Cirrad(CMk_part)) based on the abacus of correlation cases and the respective calculated third sign and,
* determining a sense of variation of at least one parameter of interest with an amplitude and or a phase of a thermal or acoustic signal based on at least one determined correlation case.

6. A computer program comprising instructions which, when executed, cause the non-invasive sensor of claim 5 to execute the steps of the process of any of claims 1 to 4.

**Patentansprüche**

1. Verfahren zur Analyse von mindestens einem Parameter von Interesse in einem Ziel-Mehrschichtmedium (2) mit einem nicht-invasiven Sensor (1) auf der Grundlage der photoakustischen oder photothermischen Detektion, wobei das Verfahren folgendes umfasst:

a) Bereitstellen eines nicht-invasiven Sensors, der Folgendes umfasst:

- eine Lichtquelle (lla), deren Irradiationskonfiguration (Cirrad) einstellbar ist, wobei diese Irradiationskonfiguration mindestens eine Wellenlänge ($\lambda$) und mindestens eine jeweilige zugehörige Intensitätsmodulationsfrequenz (fmod) umfasst,
- eine Detektionszelle (12), ausgelegt zum Erfassen eines akustischen oder thermischen Signals,
- ein Signalverarbeitungsmodul (13),
- ein Adaptationsmodul (14), das einen Prozessor und einen Speicher umfasst, **dadurch gekennzeichnet, dass** im Speicher ein Abakus der Korrelationsfälle gespeichert ist, wobei der Abakus der Korrelationsfälle für jedes von mehreren Paaren, die jeweils eine der mehreren Modellkonfigurationen (CMk) des Ziel-Mehrschichtmediums (2) und jeweils eine der mehreren Irradiationskonfigurationen (Cirrad) der Lichtquelle (lla) umfassen, mindestens einen zugehörigen Korrelationsfall enthält, der für mindestens eine Wellenlänge der Irradiationskonfiguration und die jeweils zugeordnete Modulationsfrequenz (fmod) Folgendes umfasst:
- ein erstes Vorzeichen, das das Vorzeichen des Korrelationskoeffizienten (Rparam/amp($\lambda$, fmod)) zwischen der Amplitude der detektierten Welle bei der jeweiligen Wellenlänge und zugehörigen Modulationsfrequenz und dem Parameter von Interesse ist,
- ein zweites Vorzeichen, das das Vorzeichen des Korrelationskoeffizienten (Rparam/ph($\lambda$, fmod)) zwischen der Phase der detektierten Welle bei der jeweiligen Wellenlänge und zugehörigen Modulationsfrequenz und dem Parameter von Interesse ist, und

- ein drittes Vorzeichen, das das Vorzeichen des Korrelationskoeffizienten (Rph/amp($\lambda$, fmod)) zwischen der Amplitude und der Phase der detektierten Welle bei der jeweiligen Wellenlänge und zugehörigen Modulationsfrequenz ist; das Verfahren umfasst ferner

b) Bestimmen einer bestimmten Modellkonfiguration (CMk_part) des Ziel-Mehrschichtmediums (2);

c) Festlegen mit dem Adaptationsmodul (14) einer bestimmten Irradiationskonfiguration (Cirrad(CMk_part)) für die anschließende Bestrahlung auf der Grundlage des Abakus der Korrelationsfälle und der bestimmten Modellkonfiguration;

d) Durchführen einer Vielzahl von Bestrahlungen des Ziel-Mehrschichtmediums (2) während eines Bestrahlungszeitintervalls (Tirrad) mit der Lichtquelle (IIa), die mit der in Schritt c) bestimmten Irradiationskonfiguration (Cirrad(CMk _part)) konfiguriert ist, und Erfassen des jeweils als Reaktion auf die Bestrahlung erzeugten Signals mit der Detektionszelle (12) sowie Bestimmen der jeweiligen Amplitude und der jeweiligen Phase des genannten Signals bei mindestens einer Wellenlänge und bei mindestens einer jeweils zugehörigen Modulationsfrequenz der bestimmten Irradiationskonfiguration mittels des Signalverarbeitungsmoduls (13);

e) Mit dem Adaptationsmodul (14) wird das dritte Vorzeichen eines Korrelationsfalls berechnet, das mit mindestens einer Wellenlänge der bestimmten Irradiationskonfiguration (Cirrad(CMk_part)) und der jeweils zugehörigen Modulationsfrequenz assoziiert ist, basierend auf den ermittelten Amplituden und Phasen;

f) Bestimmen mit dem Adaptationsmodul (14) eines Korrelationsfalls, der mit mindestens einer Wellenlänge und der jeweiligen Modulationsfrequenz der bestimmten Irradiationskonfiguration (Cirrad(CMk_part)) assoziiert ist, auf Grundlage des Abakus der Korrelationsfälle und des jeweils berechneten dritten Vorzeichens;

g) Bestimmen mit dem Adaptationsmodul (14) der Richtung der Veränderung von mindestens einem interessierenden Parameter anhand der Amplitude und/oder der Phase des thermischen oder akustischen Signals, basierend auf mindestens einem in f) bestimmten zugehörigen Korrelationsfall.

2. Verfahren zur Analyse von mindestens einem interessierenden Parameter in einem Ziel-Mehrschichtmedium (2) gemäß Anspruch 1, das nach g) folgendes umfasst: Bestimmen der zeitlichen Richtung der Veränderung von mindestens einem interessierenden Parameter basierend auf dem Signal, das als Reaktion auf eine zusätzliche Bestrahlung des Ziel-Mehrschichtmediums (2) mit der bestimmten Irradiationskonfiguration erzeugt wurde, und auf der in g) bestimmten Richtung der Veränderung in Bezug auf die Amplitude und/oder Phase des thermischen oder akustischen Signals.

3. Verfahren zur Analyse von mindestens einem interessierenden Parameter in einem Ziel-Mehrschichtmedium (2) gemäß Anspruch 1 oder Anspruch 2, das nach f) und vor g) mindestens eine Iteration von a), b), c), d), e) und f) umfasst;

4. Verfahren zur Analyse von mindestens einem interessierenden Parameter in einem Ziel-Mehrschichtmedium (2) gemäß einem der Ansprüche 1 bis 3, das vor a) Folgendes umfasst:

i.- Durchführen einer induzierten kontrollierten Veränderung des interessierenden Parameters,

ii- Durchführen eines zusätzlichen Schritts c),

iii- Durchführen eines zusätzlichen Schritts e),

iv- Mit dem Adaptationsmodul (14) werden das erste und das zweite Vorzeichen eines Korrelationsfalls berechnet, der mit mindestens einer der in Schritt ii) bestimmten Wellenlängen und jeweils mindestens einer der zugehörigen Modulationsfrequenzen der betreffenden Irradiationskonfiguration (Cirrad(CMk_part)) assoziiert ist, basierend auf den in Schritt iii) bestimmten jeweiligen Amplituden und Phasen,

v- Durchführen eines zusätzlichen Schritts f),

und Bereitstellung des in v. bestimmten Korrelationsfalls als zusätzliche Eingabe für a).

5. Nicht-invasiver Sensor (1) zur Analyse von mindestens einem interessierenden Parameter in einem Ziel-Mehrschichtmedium (2) auf Basis photoakustischer oder photothermischer Detektion, umfassend:

- eine Lichtquelle (IIa), deren Irradiationskonfiguration (Cirrad) abstimmbar ist, wobei diese Irradiationskonfiguration mindestens eine Wellenlänge ($\lambda$) und jeweils mindestens eine Intensitätsmodulationsfrequenz (fmod) aufweist;

- eine Detektionszelle (12), konfiguriert zur Detektion eines akustischen oder thermischen Signals;

- ein Signalverarbeitungsmodul (13);

- ein Adaptationsmodul (14), umfassend:

1) einen Prozessor,

2) ein Speicher, **dadurch gekennzeichnet, dass** der Speicher einen Abakus der Korrelationsfälle umfasst, wobei der Abakus der Korrelationsfälle für jedes von mehreren Paaren, die jeweils eine Modellkonfiguration (CMk) des Ziel-Mehrschichtmediums (2) und jeweils eine Irradiationskonfiguration (Cirrad) der Lichtquelle (IIa) umfassen, mindestens einen zugehörigen Korrelationsfall enthält, der für mindestens eine Wellenlänge ($\lambda$) der Irradiationskonfiguration und die jeweilige zugehörige Modulationsfrequenz (fmod) Folgendes enthält:

- ein erstes Vorzeichen, nämlich das Vorzeichen des Korrelationskoeffizienten (Rparam/amp($\lambda$, fmod)) zwischen der Amplitude der detektierten Welle bei der jeweiligen Wellenlänge und der zugehörigen Modulationsfrequenz und dem Parameter von Interesse,
- ein zweites Vorzeichen, nämlich das Vorzeichen des Korrelationskoeffizienten (Rparam/ph($\lambda$, fmod)) zwischen der Phase der detektierten Welle bei der jeweiligen Wellenlänge und der zugehörigen Modulationsfrequenz und dem Parameter von Interesse, und
- ein drittes Vorzeichen, nämlich das Vorzeichen des Korrelationskoeffizienten (Rph/amp($\lambda$, fmod)) zwischen Amplitude und Phase der detektierten Welle bei der jeweiligen Wellenlänge und der zugehörigen Modulationsfrequenz

Das Adaptationsmodul ist konfiguriert für:

* Bestimmen einer bestimmten Modellkonfiguration (CMk_part) des Ziel-Mehrschichtmediums (2),
*Bestimmen einer bestimmten Irradiationskonfiguration (Cirrad(CMk_part)) für die anschließende Bestrahlung auf der Grundlage des Abakus der Korrelationsfälle und der bestimmten Modellkonfiguration,
*Berechnen eines dritten Vorzeichens eines Korrelationsfalls, der mit mindestens einer der mindestens einen Wellenlänge und der jeweiligen zugehörigen Modulationsfrequenz der bestimmten Irradiationskonfiguration (Cirrad(CMk_part)) assoziiert ist, basierend auf den ermittelten jeweiligen Amplituden und Phasen,
*Bestimmen eines Korrelationsfalls, der mit mindestens einer der mindestens einen Wellenlänge und der jeweiligen zugehörigen Modulationsfrequenz einer bestimmten Irradiationskonfiguration (Cirrad(CMk _part)) assoziiert ist, basierend auf dem Abakus der Korrelationsfälle und dem jeweils berechneten dritten Vorzeichen,
* Bestimmen einer Richtung der Variation von mindestens einem Parameter von Interesse in Bezug auf Amplitude und/oder Phase eines thermischen oder akustischen Signals basierend auf mindestens einem bestimmten Korrelationsfall.

6. Ein Computerprogramm, umfassend Anweisungen, die, wenn sie ausgeführt werden, den nicht-invasiven Sensor des Anspruchs 5 dazu veranlassen, die Schritte des Verfahrens eines der Ansprüche 1 bis 4 auszuführen.


**Revendications**

1. Procédé d'analyse d'au moins un paramètre d'intérêt dans un milieu multicouche cible (2) à l'aide d'un capteur non invasif (1) basé sur la détection photoacoustique ou photothermique, le procédé comprenant :

a) la mise en place d'un capteur non invasif comprenant :

- une source lumineuse (11a) dont la configuration d'irradiation (Cirrad) est réglable, cette configuration d'irradiation comprenant au moins une longueur d'onde ($\lambda$) et au moins une fréquence de modulation d'intensité (fmod) associée respective,
- une cellule de détection (12) configurée pour détecter un signal acoustique ou thermique,
- un module de traitement du signal (13),
- un module d'adaptation (14) comprenant un processeur et une mémoire, **caractérisé en ce que** la mémoire stocke un abac de cas de corrélation, l'abac de cas de corrélation comprenant, pour chacune d'une pluralité de paires comprenant l'une d'une pluralité de configurations modèles (CMk) du milieu multicouche cible (2) et l'une d'une pluralité de configurations d'irradiation (Cirrad) de la source lumineuse (11a), au moins un cas de corrélation associé qui comprend, pour au moins une longueur d'onde de la configuration d'irradiation et une fréquence de modulation associée respective (fmod) :

- un premier signe qui est le signe du coefficient de corrélation (Rparam/amp($\lambda$, fmod)) entre l'amplitude de l'onde détectée à la longueur d'onde et à la fréquence de modulation associées respectives et le paramètre d'intérêt,

- un deuxième signe qui est le signe du coefficient de corrélation (Rparam/ph($\lambda$, fmod)) entre la phase de l'onde détectée à la longueur d'onde respective et à la fréquence de modulation associée et le paramètre d'intérêt, et

- un troisième signe qui est le signe du coefficient de corrélation (Rph/amp($\lambda$, fmod)) entre l'amplitude et la phase de l'onde détectée à la longueur d'onde respective et à la fréquence de modulation associée ; le procédé comprenant en outre

b) déterminer une configuration particulière du modèle (CMk_part) du milieu multicouche cible (2) ;

c) déterminer, à l'aide du module d'adaptation (14), une configuration d'irradiation particulière (Cirrad(CMk_part)) pour l'irradiation ultérieure, sur la base du tableau des cas de corrélation et de la configuration de modèle particulière ;

d) effectuer une pluralité d'irradiations du milieu multicouche cible (2) pendant un intervalle de temps d'irradiation (Tirrad) avec la source lumineuse (11a) configurée selon la configuration d'irradiation particulière (Cirrad(CMk_part)) déterminée à l'étape c) et détecter le signal respectif généré en réponse à l'irradiation à l'aide de la cellule de détection (12) et déterminer une amplitude respective et une phase respective dudit signal à au moins une longueur d'onde et au moins une fréquence de modulation associée respective de la configuration d'irradiation particulière à l'aide du module de traitement du signal (13) ;

e) à l'aide du module d'adaptation (14), calculer le troisième signe d'un cas de corrélation associé à au moins l'une de la au moins une longueur d'onde et de la au moins une fréquence de modulation associée respective de l' e la configuration d'irradiation particulière (Cirrad(CMk_part)) sur la base des amplitudes et des phases respectives déterminées ;

f) déterminer, à l'aide du module d'adaptation (14), un cas de corrélation associé à au moins l'une de la au moins une longueur d'onde et de la fréquence de modulation respective de la configuration d'irradiation particulière (Cirrad(CMk_part)), sur la base du tableau des cas de corrélation et du troisième signe calculé correspondant ;

g) déterminer, à l'aide du module d'adaptation (14), le sens de variation d'au moins un paramètre d'intérêt avec une amplitude et/ou une phase du signal thermique ou acoustique sur la base du au moins un cas de corrélation associé déterminé à l'étape f).

2. Procédé d'analyse d'au moins un paramètre d'intérêt dans un milieu multicouche cible (2) selon la revendication 1, comprenant après g) : la détermination du sens de variation dans le temps d'au moins un paramètre d'intérêt sur la base du signal généré en réponse à une irradiation supplémentaire du milieu multicouche cible (2) avec la configuration d'irradiation particulière et le sens de variation avec l'amplitude et/ou la phase du signal thermique ou acoustique déterminé en g).

3. Procédé d'analyse d'au moins un paramètre d'intérêt dans un milieu multicouche cible (2) selon la revendication 1 ou la revendication 2, comprenant, après f) et avant g), au moins une itération des étapes a), b), c), d), e) et f) ;

4. Procédé d'analyse d'au moins un paramètre d'intérêt dans un milieu multicouche cible (2) selon l'une quelconque des revendications 1 à 3, comprenant avant a) :

i- la réalisation d'une variation contrôlée induite du paramètre d'intérêt,

ii- effectuer une étape supplémentaire c),

iii- effectuer une étape e) supplémentaire,

iv- à l'aide du module d'adaptation (14), calculer un premier signe et un second signe d'un cas de corrélation associé à au moins l'une parmi la au moins une longueur d'onde et la au moins une fréquence de modulation associée respective de la configuration d'irradiation particulière (Cirrad(CMk_part)) déterminée à l'étape ii) sur la base des amplitudes et des phases respectives déterminées à l'étape iii),

v- effectuer une étape supplémentaire f),

et fournir comme entrée supplémentaire pour a) le cas de corrélation déterminé à l'étape v.

5. Capteur non invasif (1) destiné à analyser au moins un paramètre d'intérêt dans un milieu multicouche cible (2) sur la base d'une détection photoacoustique ou photothermique, comprenant :

- une source lumineuse (11a) dont la configuration d'irradiation (Cirrad) est réglable, cette configuration d'irradiation comprenant au moins une longueur d'onde ($\lambda$) et au moins une fréquence de modulation d'intensité (fmod) respective ;

- une cellule de détection (12) configurée pour détecter un signal acoustique ou thermique ;
- un module de traitement du signal (13) ;
- un module d'adaptation (14) comprenant :

1) un processeur,
2) une mémoire, **caractérisée en ce que** la mémoire comprend un abacus de cas de corrélation, l'abacus de cas de corrélation comprenant, pour chacune d'une pluralité de paires comprenant l'une d'une pluralité de configurations modèles (CMk) du milieu multicouche cible (2) et l'une d'une pluralité de configurations d'irradiation (Cirrad) de la source lumineuse (11a), au moins un cas de corrélation associé qui comprend, pour au moins une longueur d'onde ($\lambda$) de la configuration d'irradiation et une fréquence de modulation associée respective (fmod) :

- un premier signe qui est le signe du coefficient de corrélation (Rparam/amp($\lambda$, fmod)) entre l'amplitude de l'onde détectée à la longueur d'onde respective et à la fréquence de modulation associée et le paramètre d'intérêt,
- un deuxième signe qui est le signe du coefficient de corrélation (Rparam/ph($\lambda$, fmod)) entre la phase de l'onde détectée à la longueur d'onde respective et à la fréquence de modulation associée et le paramètre d'intérêt, et
- un troisième signe qui est le signe du coefficient de corrélation (Rph/amp($\lambda$, fmod)) entre l'amplitude et la phase de l'onde détectée à la longueur d'onde respective et à la fréquence de modulation associée

le module d'adaptation étant configuré pour :

* déterminer une configuration de modèle particulière (CMk_part) du milieu multicouche cible (2), * déterminer une configuration d'irradiation particulière (Cirrad(CMk_part)) pour une irradiation ultérieure sur la base de l'abaque des cas de corrélation et de la configuration de modèle particulière,
* calculer un premier et/ou un deuxième et/ou un troisième signe d'un cas de corrélation associé à au moins l'une de la au moins une longueur d'onde et d'une fréquence de modulation associée respective de la configuration d'irradiation particulière (Cirrad(CMk_part)) sur la base des amplitudes et des phases respectives déterminées,
*déterminer un cas de corrélation associé à au moins l'une parmi la au moins une longueur d'onde et une fréquence de modulation associée respective d'une configuration d'irradiation particulière (Cirrad(CMk_part)) sur la base du tableau des cas de corrélation et d'un troisième signe calculé respectif, et
* déterminer un sens de variation d'au moins un paramètre d'intérêt avec une amplitude et/ou une phase d'un signal thermique ou acoustique sur la base d'au moins un cas de corrélation déterminé.

6. Programme informatique comprenant des instructions qui, lorsqu'elles sont exécutées, amènent le capteur non invasif selon la revendication 5 à exécuter les étapes du procédé selon l'une quelconque des revendications 1 à 4.

Fig. 1

Fig. 2

Fig. 3a

Fig. 3b

Zoom fnod1_amp

fnod1_amp ⊂ [500; 535 Hz]

Fig. 3c

Fig. 4a

Fig. 4b

Fig. 4c

Fig. 5

EP 4 368 097 B1

| MODULATION FREQUENCY (Hz) | Epidermis water content (%) | Stratum corneum water content (%) = 20% | | | | | | | | | |
| | | Stratum corneum thickness = 18 micrometers | | | | | Stratum corneum thickness = 35 micrometers | | | | |
| | | 40 | 50 | 60 | 70 | 80 | 40 | 50 | 60 | 70 | 80 |
| | 50 | Zone 1 | Zone 4 | Zone 4 | Zone 4 | Zone 4 | Zone 1 | Zone 1 | Zone 1 | Zone 1 | Zone 1 |
| | 60 | Zone 1 | Zone 1 | Zone 1 | Zone 4 | Zone 4 | Zone 1 | Zone 1 | Zone 1 | Zone 1 | Zone 1 |
| | 100 | Zone 1 | Zone 1 | Zone 1 | Zone 1 | Zone 1 | Zone 1 | Zone 1 | Zone 1 | Zone 1 | Zone 1 |
| | 200 | Zone 1 | Zone 1 | Zone 1 | Zone 1 | Zone 1 | Zone 2 | Zone 2 | Zone 2 | Zone 2 | Zone 2 |
| | 300 | Zone 1 | Zone 1 | Zone 1 | Zone 1 | Zone 1 | Zone 2 | Zone 2 | Zone 2 | Zone 2 | Zone 2 |
| | 400 | Zone 1 | Zone 1 | Zone 1 | Zone 1 | Zone 1 | Zone 3 | Zone 2 | Zone 2 | Zone 2 | Zone 2 |
| | 500 | Zone 2 | Zone 1 | Zone 1 | Zone 1 | Zone 1 | Zone 3 | Zone 3 | Zone 3 | Zone 3 | Zone 3 |
| | 600 | Zone 2 | Zone 2 | Zone 2 | Zone 2 | Zone 2 | Zone 3 | Zone 3 | Zone 3 | Zone 3 | Zone 3 |
| | 700 | Zone 2 | Zone 2 | Zone 2 | Zone 2 | Zone 2 | Zone 3 | Zone 3 | Zone 3 | Zone 3 | Zone 3 |
| | 800 | Zone 2 | Zone 2 | Zone 2 | Zone 2 | Zone 2 | Zone 3 | Zone 3 | Zone 3 | Zone 3 | Zone 3 |
| | 900 | Zone 2 | Zone 2 | Zone 2 | Zone 2 | Zone 2 | Zone 4 | Zone 4 | Zone 4 | Zone 4 | Zone 4 |
| | 1000 | Zone 2 | Zone 2 | Zone 2 | Zone 2 | Zone 2 | Zone 4 | Zone 4 | Zone 4 | Zone 4 | Zone 4 |
| | 2000 | Zone 3 | Zone 3 | Zone 3 | Zone 3 | Zone 3 | Zone 1 | Zone 1 | Zone 1 | Zone 1 | Zone 1 |
| | 3000 | Zone 4 | Zone 3 | Zone 3 | Zone 3 | Zone 3 | Zone 2 | Zone 2 | Zone 2 | Zone 2 | Zone 2 |
| | 4000 | Zone 4 | Zone 4 | Zone 4 | Zone 4 | Zone 4 | Zone 3 | Zone 3 | Zone 3 | Zone 3 | Zone 3 |

Figure 6

physiological
database

61 : choice of model
configuration
CMk_part

Abacus of
correlation cases

62 : Choice of
irradiation configuration
Cirrad(CMk_part)

63 : Repeat p times :
- irradiation with Cirrad(CMk_part)
- and detection of amplitude and
phase of the generated wave

64 : Determination of
sgn(Rph/amp)

65 : Determination of the
correlation case

66 : determination of the sense of
variation of the parameter of interest
with amplitude and/or phase

Figure 7

physiological database

61 : choice of model configuration CMk_part

Abacus of correlation cases

62 : Choice of irradiation configuration Cirrad(CMk_part)

63 : Repeat p times:
- irradiation with Cirrad(CMk_part)
- and detection of amplitude and phase of the generated wave

64 : Determination of sgn(Rph/amp)

65 : Determination of the correlation case

66 : determination of the sense of variation of the parameter of interest with amplitude and/or phase

Figure 8

physiological database

61

81 : induced controlled variation of parameter of interest

Abacus of correlation cases

62

84 : determination of sgn(Rph/param) and sgn(Ramp/param)

63

64

65 : Determination of the correlation case

66 : determination of the sense of variation of the parameter of interest with amplitude and/or phase

Figure 9

## REFERENCES CITED IN THE DESCRIPTION

**Patent documents cited in the description**

- CN 109662720 A

- EP 22173132 A

**Non-patent literature cited in the description**

- **ROSENCWAIG, A.** ; **GERSHO, A.** Theory of the photoacoustic effect with solids. *Journal of Applied Physics*, 1976, vol. 47, 64
- **HU, H.** ; **WANG, X.** ; **XU, X.** Generalized theory of the photoacoustic effect in a multilayer material.. *Journal of Applied Physics*, 1999, vol. 86, 3953-3958
- **C. A. BENNETT** ; **R. R. PATTY**. Thermal wave interferometry: a potential application of the photoacoustic effect. *Appl. Opt.*, 1982, vol. 21, 49-54
- **ANDREAS MANDELIS**. Theory of photothermal-wave diffraction and interference in condensed media. *J. Opt. Soc. Am. A*, 1989, vol. 6, 298-308
- **ANDREAS MANDELIS** ; **KWAN F. LEUNG**. Photothermal-wave diffraction and interference in condensed media: experimental evidence in aluminum. *J. Opt. Soc. Am. A*, 1991, vol. 8, 186-200
- **HU, HANPING et al.** Generalized theory of the photoacoustic effect in a multilayer material.. *Journal of Applied Physics*, 1999, vol. 86, 3953-3958
- **CAO, J.** Interferential formulization and interpretation of the photoacoustic effect in multi-layered cells. *Journal of Physics D: Applied Physics*, 2000, vol. 33, 200